# EUROPEAN PATENT SPECIFICATION

(11) **EP 1 860 950 B1**
(45) Date of publication and mention of the grant of the patent: **19.04.2017**
(21) Application number: 06737248.2
(22) Date of filing: 03.03.2006
(51) Int. Cl.: A01N 63/00, C12N 5/00

(54) **ADULT PANCREATIC DERIVED STROMAL CELLS**
VOM PANKREAS ERWACHSENER ABGELEITETE STROMAZELLEN
CELLULES STROMALES ADULTES D'ORIGINE PANCREATIQUE

(30) Priority: 04.03.2005 US 658929 P
(43) Date of publication of application: 05.12.2007
(73) Proprietor: Lifescan, Inc., Milpitas, CA 95035-6312 (US)
(72) Inventor: O'NEIL, John, Belle Mead, NJ 08502 (US); XU, Jean, Hillsborough, NJ 08844 (US); REZANIA, Alireza, Hillsborough, NJ 08844 (US)
(74) Representative: Goodfellow, Hugh Robin
(86) International application number: PCT/US2006/008055
(87) International publication number: WO 2006/094286

(56) References cited:
- WO-A-03/026584
- SEABERG R ET AL: "Clonal identification of multipotent precursors from adult mouse pancreas that generate neural and pancreatic lineages" NATURE BIOTECHNOLOGY, NATURE PUBLISHING GROUP, NEW YORK, NY, US, vol. 22, no. 9, 1 September 2004 (2004-09-01), pages 1115-1124, XP002987994 ISSN: 1087-0156
- CORNELIUS J G ET AL: "In vitro-generation of islets in long-term cultures of pluripotent stem cells from adult mouse pancreas" HORMONE AND METABOLIC RESEARCH, THIEME-STRATTON, STUTTGART, DE, vol. 29, no. 6, 1 January 1997 (1997-01-01), pages 271-277, XP000864742 ISSN: 0018-5043
- RAMIYA VIJAYAKUMAR K ET AL: "Reversal of insulin-dependent diabetes using islets generated in vitro from pancreatic stem cells" NATURE MEDICINE, NATURE PUBLISHING GROUP, NEW YORK, NY, US, vol. 6, no. 3, 1 March 2000 (2000-03-01), pages 278-282, XP000864764 ISSN: 1078-8956
- DAVANI BEHROUS ET AL: "Human islet-derived precursor cells are mesenchymal stromal cells that differentiate and mature to hormone-expressing cells in vivo" STEM CELLS, ALPHAMED PRESS, DAYTON, OH, US, vol. 25, no. 12, 1 January 2007 (2007-01-01), pages 3215-3222, XP009109356 ISSN: 1066-5099
- PECK AMMON B ET AL: "In vitro-generation of surrogate islets from adult stem cells." TRANSPLANT IMMUNOLOGY APR 2004, vol. 12, no. 3-4, April 2004 (2004-04), pages 259-272, XP002506321 ISSN: 0966-3274
- HOEM ET AL.: 'Nonadhesive organ culture of human exocrine pancreatic cells with their stroma' PANCREAS vol. 25, no. 1, 2002, pages 71 - 77, XP008091282
- YECHOOR ET AL.: 'Gene Therapy Progress and Prospects: Gene Therapy for diabetes mellitus' GENE THERAPY vol. 12, 2005, pages 101 - 107, XP003020076
- JUN ET AL.: 'Approaches for the Cure of Type i Diabetes by Cellular and Gene Therapy' CURRENT GENE THERAPY vol. 5, 01 January 2005, pages 249 - 262, XP008073149

## Description

### FIELD OF THE INVENTION

This invention relates to an expandable population of adult pancreatic cells that can be differentiated into a β-cell lineage. This invention also provides methods for isolating and expanding such adult pancreatic cells, as well as related methods and compositions for utilizing such cells in the therapeutic treatment of diabetes.

### BACKGROUND OF THE INVENTION

Loss of organ function can result from congenital defects, injury or disease. One example of a disease causing loss of organ function is diabetes mellitus, or diabetes. Most cases of diabetes fall into two clinical types: Type 1, also known as juvenile-onset diabetes or insulin dependent diabetes mellitus (IDDM), and Type 2, also known as adult-onset diabetes. Each type has a different prognosis, treatment, and cause. Both classes are characterized by the patient's inability to regulate their blood glucose levels. As a consequence, blood glucose levels rise to high values because glucose cannot enter cells to meet metabolic demands. This inability to properly metabolize blood sugar causes a complex series of early and late-stage symptomologies, beginning with, for example, hyperglycemia, abnormal hunger, thirst, polyuria, and glycouria, and then escalating to, for example, neuropathy, macro-vascular disease, and micro-vascular disease.

A common method of treatment of Type 1 diabetes involves the exogenous administration of insulin, typically by injection with either a syringe or a pump. This method does not completely normalize blood glucose levels and is often associated with an increased risk of hypoglycemia. More effective glycemic control can be achieved if the function of the pancreas can be restored or rejuvenated via transplantation or cell-based therapies.

It has been documented that progenitor cells, derived from fetal or embryonic tissues, have the potential to differentiate into a pancreatic hormone-producing cell. See, for example, U.S. Patent 6,436,704, WO03/062405, WO02/092756 and EP 0 363 125 A2, which report the potential of human fetal and embryonic derived cells to differentiate into a β-cell lineage. It is important to note from these publications, however, that human embryonic cells often require a feeder layer for expansion and maintenance of pluripotency, which does not allow for facile scale up of cells and an eventual cell therapy for treating diabetes.

It has also been documented that progenitor cells derived from adult tissues are capable of differentiation into a pancreatic β-cell phenotype. See, for example, WO2004/087885 A2, Hess et al. (Nature Biotechnology 21, 763 - 770, 2003), and Ianus et al. (J. Clin. Invest. 111: 843-850, 2003), which report the capacity of adult bone marrow-derived cells (mesenchymal and hematopoetic cells) to differentiate into cells having characteristics of a pancreatic β-cell *in vitro,* or secrete trophic factors that help regenerate a damaged pancreas *in vivo.*

Among other sources of progenitor cells that can be differentiated into pancreatic cells include rodent liver oval stem cells (WO03/033697) and post-partum placenta (U.S. Published Application 2004/0161419 A1).

The endocrine cells of the islets of Langerhans, including β-cells, are constantly turning over by processes of apoptosis and the proliferation of new islet cells (neogenesis). As such, the pancreas is thought to be a source of progenitor cells that are capable of differentiating into pancreatic hormone producing cells. There are three distinct tissue types, isolated from a pancreas, that are a potential source of pancreatic progenitor cells: an islet rich fraction, a ductal cell rich fraction, and an acinar cell rich fraction.

Isolation of progenitor cells or partially differentiated cells from crude pancreatic tissue extracts may be achieved using antibodies raised against cell surface markers. For example, U.S. Published Application 2004/0241761 discloses isolation of murine cells that were positive for ErbB2, ErbB3, ErbB4, Msx-2 and PDX-1 (Cell 1, **Table I** hereinbelow), and positive for insulin expression.

Gershengorn et al. (Science 306: 2261-2264, 2004) teach the production of proliferating cells that were able to form islet-like cell aggregates. The cells were derived from a heterogeneous population of adherent cells that emerged from the culture of isolated human pancreatic islets *in vitro.* The isolated islets of Langerhans were initially seeded onto tissue culture dishes and cultured in medium containing 10% serum. Fibroblast-like cells were observed to migrate out of the cultured islets and form a monolayer. These cells expressed Nestin, smooth muscle actin and vimentin (Cell 2, **Table I** hereinbelow).

Pancreatic progenitor cells may also arise from the culture of pancreatic islet and ductal tissue that has been dissociated into single cells, as disclosed by Seaberg et al. (Nature Biotechnology 22: 1115 - 1124, 2004). The murine progenitor cells disclosed by Seaberg *et al.* expressed Nestin during proliferation (Cell 3, **Table I** hereinbelow).

U.S. Published Application 2003/0082155 discloses methods to isolate and identify a population of cells from the islets of Langerhans of human pancreas, which have the functional and molecular characteristics of stem cells. In particular, these cells were characterized by Nestin-positive staining, Nestin gene expression, GLP-1R-positive staining, GLP- 1R gene expression, ABCG2 positive staining, ABCG2 gene expression, Oct3/4 positive staining, Oct3/4 gene expression, latrophilin (type 2) positive staining, latrophilin (type 2) gene expression, Hes-1 positive staining, Hes-1 gene expression, Integrin subunits α6 and β1 positive staining, Integrin subunits α6 and β1 gene expression, C-kit positive staining, C-kit gene expression, MDR-1 positive staining, MDR-1 gene expression, SST-R, 2, 3, 4 positive staining, SST-R, 2, 3, 4 gene expression, SUR-1 positive staining, SUR-1 gene expression, Kir 6.2 positive staining, Kir 6.2 gene expression, CD34 negative staining, CD45 negative staining, CD133 negative staining, MHC class I negative staining, MHC class II negative staining, cytokeratin-19 negative staining, long-term proliferation in culture, and the ability to differentiate into pseudo-islets in culture (Cell 4, **Table I** hereinbelow).

In another approach, as disclosed in U.S. Patent 5,834,308, U.S. Patent 6,001,647 and U.S. Patent 6,703,017, crude preparations of islet cultures from NOD mice may be used to establish epithelial-like cultures, which can be maintained in growing cultures for greater than 1 year and which appear to demonstrate the ability to differentiate into islet-like clusters, capable of secreting insulin. (Cell 5, **Table I** hereinbelow).

Islet-like structures may be generated from fractions of digested human pancreata enriched for ductal tissue, as disclosed in Bonner-Weir et al. (Proc Nat Acad Sci 97: 7999-8004, 2000) and U.S. Patent 6,815,203 B1. Islet-like clusters disclosed in these publications stained positive for cytokeratin 19 and showed immunoreactivity for insulin. (Cell 6, **Table I** hereinbelow).

WO2004/01162 discloses the generation of insulin negative adherent cells from human pancreatic ductal fragments. (Cell 7, **Table I** hereinbelow).

WO03/102134 discloses the generation of an epithelial cell positive for cytokeratin-19 from an acinar fraction of a human pancreatic digest. The cells generated are capable of limited expansion and differentiate into an insulin-producing cell in the presence of an induction media. (Cell 8, **Table I** hereinbelow).

U.S. Published Application 2004/015805 A1 reports that a subset of human pancreatic stem cells may be isolated using ligands to the cell surface marker CD56 (also known as NCAM). These cells can differentiate into insulin producing cells and insulin producing aggregates. (Cell 9, **Table I** hereinbelow).

Thus, there remains a significant need to develop culture conditions for establishing adult pancreatic derived cell lines that can be expanded to address the current clinical needs, while retaining the potential to differentiate into a β-cell lineage at late passages.

### SUMMARY OF THE INVENTION

### <INSERT CONTINUATION PAGES 4a-4c HERE>

The methods for isolating an adult pancreatic-derived cell population can include expanding the cell population for up to about 20 population doublings, while retaining the potential of the cells to differentiate into cells with characteristics of a β-cell lineage. Alternatively, the cell population can be expanded for up to about 50, or, alternatively, for up to about 70 population doublings, while retaining the potential of the cells to differentiate into cells with characteristics of a β-cell lineage.

In a method for isolating mammalian adult pancreatic-derived stromal cells according to the present invention, pancreatic cells are obtained from pancreatic tissue digests, which include differentiated pancreatic cells and undifferentiated pancreatic stromal cells. In order to select and enrich stromal cells, the cells obtained from pancreatic tissue digests are cultured in a selection media, which is a nutrient rich media containing less than 5% serum and glucose at a concentration less than 30 mM. The glucose range may also be less, for example, 6 mM or less. The culture is left undisturbed for about two to about four weeks without any media changes until cells become attached to the culture substrates.

Subsequent to the initial phase of selection and cell attachment, the selection media can be switched to a nutrient rich expansion media supplemented with serum at a concentration of greater than 1% but less than 20% and glucose at a concentration of less than 30 mM.

The resulting cell population is enriched in adult pancreatic-derived stromal cells. Such stromal cell population is negative for the following markers: NCAM, ABCG2, cytokeratin 7, 8, 18 and 19; and optionally for at least one of the following markers: EPCAM, CD 45, CD117, CD 133, CD138, or CD184, , and can be expanded for multiple generations without losing the capacity to differentiate into cells of the β-cell lineage.

The cell population enriched in adult pancreatic-derived stromal cells can be contacted, for example, with an agent (such as an antibody) that specifically recognizes a protein marker expressed by stromal cells, to identify and select adult pancreatic-derived stromal cells, thereby obtaining a substantially pure population of adult pancreatic-derived stromal cells, i.e., wherein a recognized protein marker is expressed in at least 50% of the cell population.

The isolated pure population of adult pancreatic-derived stromal cells of the invention is negative for NCAM, ABCG2, cytokeratin 7, 8, 18, and 19; and is optionally negative for at least one of the following markers: EPCAM, CD45, CD117, CD133, CD138, or CD184.

In one embodiment, the adult pancreatic-derived stromal cells isolated and expanded according to the present invention can be used in an *in vitro* assay to screen for agents that are capable of differentiating cells into the β-cell lineage.

The adult pancreatic-derived stromal cells isolated and expanded according to the present invention can be induced to differentiate into cells of the β-cell lineage under appropriate *in vitro* or *in vivo* conditions. Accordingly, the adult pancreatic-derived stromal cells selected and expanded according to the present invention, as well as the differentiated cells derived from the adult pancreatic-derived stromal cells, are useful for treating Type 1 and 2 diabetes.

### BRIEF DESCRIPTION OF THE DRAWINGS

**Figure 1** outlines the multi-stage process used to isolate the adult pancreatic-derived stromal cells of the present invention.
**Figure 2** depicts the morphology of undifferentiated adhered human adult-pancreatic derived stromal cells (100X magnification) at passage 12 grown in basal media supplemented with 5% fetal bovine serum (FBS), 1% penicillin/streptomycin (P/S), and 0.0165 mM zinc sulphate.
**Figure 3** shows the typical growth curve of adult pancreatic-derived stromal cells of the current invention. The depicted curve is for passage 10 cells.
**Figure 4** illustrates typical FACS analysis of passage 9 adult pancreatic-derived stromal cells of the current invention. a) isotype control, b) CD117 on X-axis and alkaline phosphatase on Y-axis, c) CD 105 on X-axis and CD44 on Y-axis, d) CD73 on X-axis and CD90 on Y-axis, e) EPCAM, f) ABCG2, g) Pan cytokeratin 14, 15, 16, and 19, h) Pan cytokeratin 5, 6, 8, 10, 13, and 18, i) vimentin.
**Figure 5** depicts immunostaining of passage 11 cells. Cells stained positive for vimentin, nestin, beta III tubulin, smooth muscle actin, and negative for pan cytokeratin (including CK 7 and CK 19).
**Figure 6** shows the expansion potential of an adult pancreatic-derived stromal cell population isolated according to the present invention.
**Figure 7** shows the expression of selected genes, as determined by real-time PCR with time in culture. Results shown are the expression levels of PDX-1 (Panel A), insulin (Panel B), HNF-3β (Panel C) and CK 19 (Panel D), expressed as a percentage of the levels observed in human pancreas. Time in culture, as depicted by passage number is indicated on the x-axis. Data was obtained from adult pancreatic stromal cells derived from fraction 3 of donor pancreas H4. Cells were cultured in DMEM supplemented with 2% FBS.
**Figure 8** shows the expression of selected genes, as determined by real-time PCR with time in culture. Results shown are the expression levels of PDX-1 (Panel A), insulin (Panel B), HNF-3β (Panel C) and CK 19 (Panel D), expressed as a percentage of the levels observed in human pancreas. Time in culture, as depicted by passage number is indicated on the x-axis. Data was obtained from adult pancreatic stromal cells derived from fraction 4 of donor pancreas H4. Cells were cultured in DMEM supplemented with 2% FBS.
**Figure 9** shows the expression of selected genes, as determined by real-time PCR with time in culture. Results shown are the expression levels of PDX-1 (Panel A), insulin (Panel B), HNF-3β (Panel C) and CK 19 (Panel D), expressed as a percentage of the levels observed in human pancreas. Time in culture, as depicted by passage number is indicated on the x-axis. Data was obtained from adult pancreatic stromal cells derived from fraction 5 of donor pancreas H4. Cells were cultured in DMEM supplemented with 2% FBS.
**Figure 10** shows the expression of selected genes, as determined by real-time PCR with time in culture. Results shown are the expression levels of PDX-1 (Panel A), insulin (Panel B), HNF-3β (Panel C) and CK 19 (Panel D), expressed as a percentage of the levels observed in human pancreas. Time in culture, as depicted by passage number is indicated on the x-axis. Data was obtained from adult pancreatic stromal cells derived from fraction 3 of donor pancreas H4. Cells were cultured in DMEM supplemented with 10% FBS.
**Figure 11** shows the expression of selected genes, as determined by real-time PCR with time in culture. Results shown are the expression levels of PDX-1 (Panel A), insulin (Panel B), HNF-3β (Panel C) and CK 19 (Panel D), expressed as a percentage of the levels observed in human pancreas. Time in culture, as depicted by passage number is indicated on the x-axis. Data was obtained from adult pancreatic stromal cells derived from fraction 4 of donor pancreas H4. Cells were cultured in DMEM supplemented with 10% FBS.
**Figure 12** shows the expression of selected genes, as determined by real-time PCR with time in culture. Results shown are the expression levels of PDX-1 (Panel A), insulin (Panel B), HNF-3β (Panel C) and CK 19 (Panel D), expressed as a percentage of the levels observed in human pancreas. Time in culture, as depicted by passage number is indicated on the x-axis. Data was obtained from adult pancreatic stromal cells derived from fraction 5 of donor pancreas H4. Cells were cultured in DMEM supplemented with 10% FBS.
**Figure 13** shows the expression of selected genes, as determined by real-time PCR with time in culture. Results shown are the expression levels of PDX-1 (Panel A), insulin (Panel B), HNF-3β (Panel C) and CK 19 (Panel D), expressed as a percentage of the levels observed in human pancreas. Time in culture, as depicted by passage number is indicated on the x-axis. Data was obtained from adult pancreatic stromal cells derived from fraction 3 of donor pancreas H4. Cells were cultured in CMRL supplemented with 10% FBS.
**Figure 14** shows the expression of selected genes, as determined by real-time PCR with time in culture. Results shown are the expression levels of PDX-1 (Panel A), insulin (Panel B), HNF-3β (Panel C) and CK 19 (Panel D), expressed as a percentage of the levels observed in human pancreas. Time in culture, as depicted by passage number is indicated on the x-axis. Data was obtained from adult pancreatic stromal cells derived from fraction 4 of donor pancreas H4. Cells were cultured in CMRL supplemented with 10% FBS.
**Figure 15** shows the expression of selected genes, as determined by real-time PCR with time in culture. Results shown are the expression levels of PDX-1 (Panel A), insulin (Panel B), HNF-3β (Panel C) and CK 19 (Panel D), expressed as a percentage of the levels observed in human pancreas. Time in culture, as depicted by passage number is indicated on the x-axis. Data was obtained from adult pancreatic stromal cells derived from fraction 5 of donor pancreas H4. Cells were cultured in CMRL supplemented with 10% FBS.
**Figure 16** shows the expression of selected genes, as determined by real-time PCR with time in culture. Results shown are the expression levels of PDX-1 (Panel A), insulin (Panel B), HNF-3β (Panel C) and CK 19 (Panel D), expressed as a percentage of the levels observed in human pancreas. Time in culture, as depicted by passage number is indicated on the x-axis. Data was obtained from adult pancreatic stromal cells derived from fraction 3 of donor pancreas H8. Cells were cultured in CMRL supplemented with 10% FBS.
**Figure 17** shows the expression of selected genes, as determined by real-time PCR with time in culture. Results shown are the expression levels of glucagon (Panel A), Glut-2 (Panel B), Pax-4 (Panel C) and Pax-6 (Panel D), expressed as a percentage of the levels observed in human pancreas. Time in culture, as depicted by passage number is indicated on the x-axis. Data was obtained from adult pancreatic stromal cells derived from fraction 3 of donor pancreas H8. Cells were cultured in CMRL supplemented with 10% FBS.
**Figure 18** shows the expression of selected genes, as determined by real-time PCR with time in culture. Results shown are the expression levels of somatostatin (Panel A), neuroD1 (Panel B), Nkx2.2 (Panel C) and Nkx6.1 (Panel D), expressed as a percentage of the levels observed in human pancreas. Time in culture, as depicted by passage number is indicated on the x-axis. Data was obtained from adult pancreatic stromal cells derived from fraction 3 of donor pancreas H8. Cells were cultured in CMRL supplemented with 10% FBS.
**Figure 19** shows the expression of selected genes, as determined by real-time PCR with time in culture. Results shown are the expression levels of HNF-1α (Panel A), and Sox-17 (Panel B), expressed as a percentage of the levels observed in human pancreas. Time in culture, as depicted by passage number is indicated on the x-axis. Data was obtained from adult pancreatic stromal cells derived from fraction 3 of donor pancreas H8. Cells were cultured in CMRL supplemented with 10% FBS.
**Figure 20** shows the expression of selected genes, as determined by real-time PCR following treatment.
**Figure 21** shows the expansion potential of two adult pancreatic-derived stromal cell populations isolated according to the present invention prior to, and after revival from cryopreservation.
**Figure 22** shows the effects of nutrient deprivation on the expansion potential of an adult pancreatic-derived stromal cell population isolated according to the present invention.
**Figure 23** shows the karyotype of a representative adult pancreatic-derived stromal cell population isolated according to the present invention.
**Figure 24** shows the karyotype of a representative adult pancreatic-derived stromal cell population isolated according to the present invention.
**Figure 25** shows the karyotype of a representative adult pancreatic-derived stromal cell population isolated according to the present invention.
**Figure 26** shows the karyotype of a representative adult pancreatic-derived stromal cell population isolated according to the present invention.
**Figure 27** shows the karyotype of a representative adult pancreatic-derived stromal cell population isolated according to the present invention.
**Figure 28** shows the scatter plots for genes expressed in adult pancreatic-derived stromal cells derived from fractions 3, 4, and 5 of donor pancreas H5. Pearson correlation coefficient for each comparison is also listed.
**Figure 29** shows the scatter plot for genes expressed in adult pancreatic stromal cells derived from fraction 3 of donor pancreas H5 and donor pancreas H8. Pearson correlation coefficient is also listed.

### DETAILED DESCRIPTION OF THE INVENTION

For clarity of disclosure, and not by way of limitation, the detailed description of the invention is divided into the following subsections that describe or illustrate certain features, embodiments or applications of the present invention.

### Definitions

"Pancreatic-derived cells" refer to primary pancreatic cells from mammalian species, or primary cells isolated from a mammalian pancreas, that contain acinar, duct, and islet cell types, as well as supportive cells such as stromal, stellate, neurons, vascular cells, stem cells, and progenitor cells.

"Acinar cells" refer to pancreatic cells that account for about 80% of the cells of the pancreas and produce many different enzymes including amylase, lipase, trypsin, chymotrypsin, and elastase.

"Ductal cells" refer to pancreatic cells that account for about 10% of cells of the pancreas and define the larger interlobular and intralobular ducts, as well as the smallest, intercalated ducts, that drain the pancreatic enzymes from the acini. Duct cells also produce bicarbonate and water to dilute the enzymes and alter the intestinal pH upon release into the gut from these ductal structures. Duct cells can be identified by cytokeratin 19 (CK 19).

"Islet cells" refer to endocrine cells that account for about 1 to 2% of cells of the pancreas and exist as distinct cell aggregates called islets, which contain different cell types making different hormones. β-cells account for about 60 to 80% of the islet aggregate and secrete insulin that permits glucose entry into most cells of the body. Alpha cells account for about 10 to 30% of the islet, and secrete glucagon that is released during fasting to permit glucose delivery from the liver to maintain normal blood sugar. Delta cells account for about 5 to 10% of the islet cells and secrete somatostatin that further regulates glucose levels. Pancreatic polypeptide producing cells (about 5 to 10% of the islet cells) release their hormone that alters exocrine and gastrointestinal function. There are also other islet cell types, such as endothelial cells, neuronal cells, and progenitor cells.

"Pancreatic-derived stromal cells" refer to fibroblast-like cells derived from the pancreas that have the ability to expand *in vitro* and are devoid of any of the markers that are characteristic of a pancreatic hormone-producing cell, an islet cell, a cell of β-cell lineage, or a β-cell.

β-cell lineage" refer to cells with positive gene expression for the transcription factor PDX-1 and at least one of the following transcription factors: NGN-3, Nkx2.2, Nkx6.1, NeuroD, Isl-1, HNF-3 beta, MAFA, Pax4, and Pax6.

"Pancreatic islet-like structure" refers to a three-dimensional clusters of cells derived by practicing the methods of the invention, which has the appearance of a pancreatic islet. The cells in a pancreatic islet-like structure express at least the PDX-1 gene and one hormone selected from the list glucagon, somatostatin, or insulin.

The term "hypoxic" refers to oxygen levels less than 20%, alternatively less than 10%, alternatively less than 5% but more than 1%.

The term "normoxia" refers to atmospheric oxygen levels of about 20%.

The term "substantially positive," when used in connection with a population of cells with respect to the expression of certain marker (such as a membrane receptor, cytoplasmic or nuclear protein, or a transcription factor), means that the marker is present or expressed in at least about 50%, preferably at least about 60%, and more preferably at least about 70%, of the total cell population.

The term "substantially negative," when used in connection with a population of cells with respect to the expression of certain marker (such as a membrane receptor, cytoplasmic or nuclear protein, or a transcription factor), means that the marker is not present or expressed in at about 70%, alternatively about 80%, alternatively about 90%, of the total cell population.

The term "substantially pure," when used in connection with a population of cells means that the cell of interest accounts for at least about 50%, preferably at least about 60%, and more preferably at least about 70%, and most preferably > 80% of the total cell population.

A "stem cell" as used herein refers to an undifferentiated cell that is capable of extensive propagation either *in vivo* or *ex vivo* and capable of differentiation to other cell types.

A "progenitor cell" refers to a cell that is derived from a stem cell by differentiation and is capable of further differentiation to more mature cell types. Progenitor cells typically have more restricted proliferation capacity as compared to stem cells. Alternatively a "progenitor cell" may arise from the dedifferentiation of a fully or partially differentiated cell.

"Expandable population" refers to the ability of an isolated cell population to be propagated through at least about 20, alternatively, through at least about 50, alternatively, at least about 70 population doublings in a cell culture system.

By "undifferentiated cells," when used in connection with cells isolated from a pancreas, are meant a population of pancreatic-derived cells that are substantially negative for the expression of PDX-1 or insulin.

By "differentiated cells," when used in connection with cells isolated from a pancreas, are meant a population of pancreatic-derived cells that are substantially positive for the expression of PDX-1 or insulin.

"Markers" as used herein, are nucleic acid or polypeptide molecules that are differentially expressed in a cell of interest. In this context, differential expression means an increased level of the marker for a positive marker, and a decreased level for a negative marker. The detectable level of the marker nucleic acid or polypeptide is sufficiently higher or lower in the cells of interest, compared to other cells, such that the cell of interest can be identified and distinguished from other cells, using any of a variety of methods known in the art.

"Nestin" refers to an intermediate filament protein having a sequence disclosed in Genbank Accession No. X65964, or a naturally occurring variant sequence thereof (e.g., allelic variant).

"Beta III tubulin" is part of the cytoskeletal component involved in diverse cytosolic functions, such as the generation and maintenance of cell morphology and the transport of membranous organelles. Beta III tubulin has been shown to be present in neuronal cells.

"Vimentin" is a cytoskeletal intermediate filament protein that is a general marker of cells originating in the mesenchyme.

"ABCG2" refers to ATP-binding cassette multi-drug resistance transporter G2 having a sequence disclosed in Genbank Accession No. XM 032424, or a naturally occurring variant sequence thereof (e.g., allelic variant). One of skill in the art will recognize that equivalents exist wherein the DNA sequence encoding ABCG2 may vary, but wherein the encoded amino acid sequence remains the same.

"Cluster of Differentiation" (CD) molecules are markers on the cell surface, as recognized by specific sets of antibodies, used to identify the cell type, stage of differentiation and activity state of a cell. Function and designation of CD markers are well established in the art. See, for example, The Leukocyte Antigen Fact Book, 2nd edition, Barclay, A. N. et al. Academic Press, London 1997.

"CD49b" is also refferd to as "VLA-2" and is a surface recpetor for extracellular protiens such as laminin, collagen, fibronectin, and e-cadherin. It plasy a role in both coagulation and angiogenesis and is found primarily on platelets and activated B and T cells.

"CD49e" is also referred to as "VLA-5" and serves as a receptor for fibronectin and fibrinogen. CD49e is expressed primarily on platelets, monocytes, and neutrophils.

"CD49f" is also referred to as "α6 integrin" and "VLA-6," and associates with integrin subunit beta 1 to bind laminin. CD49f is expressed primarily on epithelial cells, trophoblasts, platelets, and monocytes.

"CD90" is also referred to as "Thy-1" and is primarily expressed on hematopoietic stem cells, connective tissue cells, and various fibroblastic and stromal cells.

"CD91" is also referred to as alpha-2-marcoglobulin receptor binds to lipoproteins and also serves as a surface receptor for heat shock proteins. CD91 is primarily expressed on fibroblasts, dendritic cells, and macrophages.

"CD95" is also referred to as "FAS" or "APO-1" and is the receptor for FAS ligand. It is primarily expressed on activated B and T cells and in certain types of epithelial cells.

"CD105" is also referred to as "Endoglin" and is primarily expressed on endothelial cells, monocytes, macrophages, stromal cells, and bone marrow mesenchymal cells.

"NCAM" refers to Neural Cell Adhesion Molecule, also known as "CD56," is a Ca²⁺ independent cell adhesion molecule expressed in many tissues, including the nervous system and pancreatic tissue.

"PECAM" refers to Platelet/Endothelial Cell Adhesion Molecule and belongs to the family of cell adhesion molecules that are cell-surface glycoproteins, involved in cell-cell interactions.

"EPCAM" refers to Epithelial Cell Adhesion Molecule that is a cell surface glycoprotein involved in cell-cell interactions, primarily in epithelial cells.

"c-MET" refers to the receptor for hepatocyte growth factor.

"CD44" is also referred to as "Hermes antigen" and is the main cell surface receptor for hyaluronan. This CD is primarily expressed in most cell types, except for tissues/cells such as hepatocytes, some epithelial cells, and cardiac muscle.

"CD73" is also referred to as "ecto-5'-nucleotidase" and is primarily expressed on a subset of-B and T cells, bone marrow stromal cells, various epithelial cells, fibroblasts, and endothelial cells.

"CD81" is also referred to as Target of an Anti-Proliferative Antibody (TAPA1) and is primarily expressed on lymphocytes, endothelial and epithelial cells.

"CD 124" is also referred to as Interleukin 4 receptor and is expressed on mature B cells, T cells, hemopoietic precursors, fibroblasts, and endothelial cells.

"CD138" is also referred to as heparin sulfate proteogycan or syndecan-1 which mediates cell adhesion and is associated with later stages of B cell differentiation. It is common found on B cell precursors, plasma cells, and some epithelial cell types.

"CD151" has been implied to modify integrin function and signaling and is typically found on endothelium, platelets, megakaryocytes, and epithelium.

"CD166" is also referred to as Activated Leukocyte Cell Adhesion Molecule (ALCAM) and is involved in neuronal neurite extension, embryonic hemopoiesis, and embryonic angiogenesis. CD 166 is primarily expressed on neurons, activated T cells and monocytes, epithelial cells, and fibroblasts.

"CD184" is also referred to as CXCR4 or Stromal cell Derived Factor 1 (SDF1). It is the receptor for the CXC chemokine SDF-1 and is found in all mature blood cells, blood progenitors, endothelial and epithelial cells, astrocytes, and neurons.

"c-Kit" and "CD117" both refer to a cell surface receptor tyrosine kinase having a sequence disclosed in Genbank Accession No. X06182, or a naturally occurring variant sequence thereof (e.g., allelic variant).

"CD45" refers to the leukocyte common antigen having a sequence disclosed in Genbank Accession No. Y00638, or a naturally occurring variant sequence thereof (e.g., allelic variant).

"CD133" refers to a five-transmembrane hematopoietic stem cell antigen having a sequence disclosed in Genbank Accession No. NM 006017.

"Alkaline Phosphatase" is an enzyme made in the liver, bone, and the placenta and normally present in high concentrations in growing bone and in bile. A distinguishing characteristic of undifferentiated pluripotent stem cells is their expression of high levels of Alkaline Phosphatase (AP) on their cell surface.

By "basic defined cell culture medium" is meant a serum free or serum containing, chemically defined cell growth medium. Such medium includes, but is not limited to, Dulbecco's Modified Eagle's Medium (DMEM), alpha modified Minimum Essential Medium (alpha MMEM), Basal Medium Essential (BME), CMRL-1066, RPMI 1640, M199 medium, Ham's F10 nutrient medium and DMEM/F12. These and other useful media are available from GIBCO, Grand Island, New York, U.S.A., for example. A number of these media are reviewed in Methods in Enzymology, Volume LVIII, "Cell Culture," pp. 62-72, edited by William B. Jakoby and Ira H. Pastan, published by Academic Press Inc.

"Pharmaceutical carrier" refers to a biodegradable or non-degradable porous or nonporous matrix that can act as a carrier for transplantation of mammalian cells.

"Transplantation" as used herein, can include the steps of introducing a cell or a population of cells or tissue into a mammal such as a human patient. "Transplantation" may also include incorporating cells or tissue into a pharmaceutical carrier, and implanting the carrier in a mammal such as a human patient.

### Isolation of Adult Pancreatic-Derived Stromal Cells

In one aspect of the present invention, pancreatic-derived stromal cells are isolated by a multi-stage method, which is depicted in **Figure 1.** This method essentially involves:
- Perfusion of a cadaver pancreas, living donor or autologous pancreas, with an enzymatic solution,
- Mechanical dissociation of the perfused pancreas,
- Layering the digested tissue over a density gradient such as polysucrose or Ficoll, followed by centrifugation to yield three distinct interfaces,
- Removing the tissues and cells at each interface,
- Culturing the tissues and cells in standard tissue culture plates in a nutrient rich selection media containing less than 5% serum,
- Leaving the culture undisturbed for about up to about two to up to about four weeks without any media changes.

Perfusion of a cadaver pancreas can be achieved with any of the enzymatic solutions well known to those skilled in the art. An example of an enzymatic solution suitable for use in the present invention contains LIBERASE HI™ (Roche - 0.5 mg/ml) and DNase I (0.2 mg/ml).

Mechanical dissociation of the pancreatic tissue can be carried out rapidly by the use of a tissue processor. Alternatively, mechanical dissociation of the pancreatic tissue can be carried out using a Ricordi Chamber or other equivalent apparatus that enables a less destructive dissociation of the tissue, compared to other procedures.

The digested pancreatic tissues are then subjected to a polysucrose or Ficoll gradient centrifugation to yield three distinct interfaces, which are enriched in cells from islets, the ductal tissue and the acinar tissue, respectively. In one embodiment, the tissues and cells are removed from each interface and cultured separately. In an alternative embodiment, the tissues and cells from all the interfaces are combined and cultured. It has been determined in accordance with the present invention that adult pancreatic-derived stromal cells can be derived from any of the three interfaces. Alternatively, the digested pancreatic tissue may be applied to a continuous density gradient whereby the islet enriched tissue, ductal tissue and acinar tissue may also be collected.

According to the present invention, the tissues and cells collected from one or more of the interfaces are cultured in a selection media to selectively enrich adult pancreatic-derived stromal cells in the cell population. The selection media is rich in nutrient and contains low levels of glucose and serum. Generally speaking, the selection media contains less than 5% serum, alternatively, about 1 to about 3% serum, alternatively, about 2% serum. The selection media contains less than 30 mM glucose. In one embodiment, the selection media is supplemented with 2% serum that is derived from the same mammalian species that the donor pancreas was harvested from. Alternatively, fetal or calf serum, serum from other species, or other serum supplements or replacements can be used to supplement the selection media. An example of a suitable selection media is composed of DMEM (5 mM glucose), 2% fetal bovine serum (FBS), 100 U/µg penicillin/streptomycin, insulin-transferrin-selenium (ITS), 2 mM L-Glutamine, 0.0165 mM ZnSO₄, and 0.38 µM 2-mercaptoethanol.

During the culture in a selection media ("the selection phase"), the cells can be cultured under hypoxic or normoxic conditions. Under hypoxic conditions, oxygen levels are lower than 20%, alternatively lower than 10%, alternatively lower than 5%, but more than 1%.

In one embodiment, the culture is maintained in the selection media undisturbed for about two to about four weeks without any media changes, at which point the cells have typically become adherent to the culture substrate used. The selection phase is considered to be complete when there is no further increase in the number of adherent cells.

It has been discovered that the methods of tissue harvest and culturing in accordance with the present invention result in a cell population enriched in adult pancreatic-derived stromal cells. By "enriched" is meant that adult pancreatic-derived stromal cells account for at least about 30%, alternatively about 40%, alternatively about 50% of all the cells in the population.

Subsequent to the initial phase of selection and cell attachment, the cells (enriched with adult pancreatic-derived stromal cells) are expanded under conditions as further described hereinbelow.

If desirable, the cell population enriched in adult pancreatic-derived stromal cells can be exposed, for example, to an agent (such as, for example, an antibody) that specifically recognizes a protein marker expressed by stromal cells, to identify and select adult pancreatic-derived stromal cells, thereby obtaining a substantially pure population of adult pancreatic-derived stromal cells.

### Characterization of The Isolated Adult Pancreatic-Derived Stromal Cells

Methods for assessing expression of protein and nucleic acid markers in cultured or isolated cells are standard in the art. These include quantitative reverse transcriptase polymerase chain reaction (RT-PCR), Northern blots, *in situ* hybridization (see, for example, Current Protocols in Molecular Biology (Ausubel et al., eds. 2001 supplement)), and immunoassays, such as, for example, immunohistochemical analysis of sectioned material, western blotting, and for markers that are accessible in intact cells, flow cytometry analysis (FACS) (see, for example, Harlow and Lane, Using Antibodies: A Laboratory Manual, New York: Cold Spring Harbor Laboratory Press (1998)).

Examples of antibodies useful for detecting certain protein markers are listed in **Table II** and **Table V.** It should be noted that other antibodies directed to the same markers that are recognized by the antibodies listed in **Table II** and **Table V,** are available or can be readily developed. Such other antibodies can also be employed for assessing expression of markers in the cells isolated in accordance with the present invention.

Characteristics of cells of the β-cell lineage are well known to those skilled in the art, and additional characteristics of the β-cell lineage continue to be identified. These characteristics can be used to confirm that the adult pancreatic-derived stromal cells isolated in accordance with the present invention have differentiated to acquire the properties characteristic of the β-cell lineage. β-cell lineage specific characteristics include the expression of one or more transcription factors such as, for example, insulin, PDX-1 (pancreatic and duodenal homeobox gene-1), NGN-3 (neurogenin-3), Hlxb9, Nkx6, Isl1, Pax6, NeuroD, Hnf1a, Hnf6, Hnf3 Beta, and MafA, among others. These transcription factors are well established in the art for identification of endocrine cells. See, for example, Edlund (Nature Reviews Genetics 3: 524-632 (2002)).

The present inventors have identified and isolated a population of adult stromal cells from the mammalian pancreas that have the capacity to proliferate for up to about 20 population doublings while maintaining the potential to differentiate into cells with characteristics of a β-cell lineage. In an alternate embodiment, the present inventors have identified and isolated a population of adult stromal cells from the mammalian pancreas that have the capacity to proliferate for up to about 50 population doublings while maintaining the potential to differentiate into cells with characteristics of a β-cell lineage. In an alternate embodiment, the present inventors have identified and isolated a population of adult stromal cells from the mammalian pancreas that have the capacity to proliferate for up to about 70 population doublings while maintaining the potential to differentiate into cells with characteristics of a β-cell lineage.

In particular, the adult pancreatic-derived stromal cells isolated in accordance with the present invention are characterized as, *inter alia,* lacking the following protein markers: NCAM, ABCG2, cytokeratin 7, 8, 18, or 19. The cells may also lack CD117. The adult pancreatic-derived stromal cells isolated in accordance with the present invention are characterized as positive for at least one of the following protein markers: CD44, CD73, CD90, or CD105, and optionally CD49b, CD49e, CD81, CD95, CD151 or CD166.

### Expansion of Adult Pancreatic-Derived Stromal Cells

In a further aspect, the present invention provides a method for expanding the adult pancreatic-derived stromal cells obtained in accordance with the present invention. As described hereinabove, pancreatic digests, which may contain a heterogeneous mixture of islets, ductal fragments and exocrine tissue, are cultured in a low serum selection media for about two to about four weeks, preferably without any media changes, to selectively enrich the desired adult pancreatic-derived stromal cells. The resulting cell population, now enriched with adult pancreatic-derived stromal cells, is then switched to a growth media to expand the adult pancreatic-derived stromal cells in the cell population.

The growth media suitable for use in the present invention can be composed of media such as, for example, DMEM containing penicillin/streptomycin (P/S) and serum at a concentration of 2% to 20%, preferably about 5%. In a specific embodiment, the growth media is composed of DMEM (2750 mg/l D-glucose; 862 mg/l glutamine), 5% fetal bovine serum, and 0.0165 mM zinc sulphate. In a specific embodiment, the growth media is supplemented with serum that is derived from the same mammalian species that the donor pancreas was harvested from. Alternatively, fetal or calf serum, or other serum supplements or replacements, such as, for example, serum albumin, may be used to supplement the growth media.

Furthermore, the adult pancreatic-derived stromal cells can be expanded by culturing in a defined growth media containing agent(s) that stimulate the proliferation of the cells of the present invention. These factors may include, for example, nicotinamide, members of TGF-β family, including TGF-β1, 2, and 3, bone morphogenic proteins (BMP-2, -4, 6, -7, -11, -12, and -13), serum albumin, fibroblast growth factor family, platelet-derived growth factor-AA, and -BB, platelet rich plasma, insulin growth factor (IGF-I, II) growth differentiation factor (GDF-5, -6, -8, -10, 11), glucagon like peptide-I and II (GLP-I and II), GLP-1 and GLP-2 mimetobody, Exendin-4, retinoic acid, parathyroid hormone, insulin, progesterone, aprotinin, hydrocortisone, ethanolamine, beta mercaptoethanol, epidermal growth factor (EGF), gastrin I and II, copper chelators such as triethylene pentamine, TGF-α, forskolin, Na-Butyrate, activin, betacellulin, insulin/transferring/selenium (ITS), hepatocyte growth factor (HGF), keratinocyte growth factor (KGF), bovine pituitary extract, islet neogenesis-associated protein (INGAP), proteasome inhibitors, notch pathway inhibitors, sonic hedgehog inhibitors, or combinations thereof. Alternatively, the adult pancreatic-derived stromal cells may be expanded by culturing in conditioned media. By "conditioned media" is meant that a population of cells is grown in a basic defined cell culture medium and contributes soluble factors to the medium. In one such use, the cells are removed from the medium, while the soluble factors the cells produce remain. This medium is then used to nourish a different population of cells.

In certain embodiments, the adult pancreatic-derived stromal cells are cultured on standard tissue culture plates. Alternatively, the culture plates may be coated with extracellular matrix proteins, such as, for example, MATRIGEL ®, growth factor reduced MATRIGEL ®, laminin, collagen, gelatin, tenascin, fibronectin, vitronectin, thrombospondin, placenta extracts or combinations thereof.

Furthermore, the adult pancreatic-derived stromal cells can be expanded *in vitro* under hypoxic or normoxic conditions.

Under the above growth conditions for expansion, the adult pancreatic-derived stromal cells isolated in accordance with the present invention can be expanded for more than about 20 population doublings, alternatively, more than about 50 population doublings, alternatively, more than about 70 population doublings while maintaining the potential to differentiate into cells with characteristics of a β-cell lineage.

### Differentiation Of Adult Pancreatic-Derived Stromal Cells

In one aspect, the present disclosure provides compositions capable of differentiating the expanded adult pancreatic-derived stromal cells of this invention into cells bearing markers characteristic of the β-cell lineage.

A basic defined culture medium, when supplied with one or more components, that support the growth of adult pancreatic-derived stromal cells and with differentiation-inducing amounts of one or more growth factors, is referred to as an "induction medium." The induction medium contains less than or equal to 2% serum. In one embodiment, fetal calf serum may be used. Alternatively, fetal bovine serum may be replaced by serum from any mammal, or by human albumin, bovine albumin or other compounds that permit or enhance differentiation of adult pancreatic-derived stromal cells to the β cell lineage. Alternatively, the induction medium may be conditioned medium.

Factors appropriate for use in the induction medium may include, for example, nicotinamide, members of TGF-β family, including TGF-β1, 2, and 3, bone morphogenic proteins (BMP-2, -4, 6, -7, -11, -12, and -13), serum albumin, fibroblast growth factor family, platelet-derived growth factor-AA, and -BB, platelet rich plasma, insulin growth factor (IGF-I, II) growth differentiation factor (GDF-5, -6, -8, - 10, 11), glucagon like peptide-I and II (GLP-I and II), GLP-1 and GLP-2 mimetobody, Exendin-4, retinoic acid, parathyroid hormone, insulin, progesterone, aprotinin, hydrocortisone, ethanolamine, beta mercaptoethanol, epidermal growth factor (EGF), gastrin I and II, copper chelators such as triethylene pentamine, TGF-α, forskolin, Na-Butyrate, activin, betacellulin, ITS, hepatocyte growth factor (HGF), keratinocyte growth factor (KGF), bovine pituitary extract, islet neogenesis-associated protein (INGAP), proteasome inhibitors, histone deacetylase inhibitors, notch pathway inhibitors, sonic hedgehog inhibitors, or combinations thereof.

In one aspect of the present invention, a combination of growth factors and chemical agents, including bFGF, Activin-A, FGF5, N2 and B27 supplements (Gibco, CA), steroid alkaloid such as, for example, cyclopamine (EMD, CA) that inhibits sonic hedgehog signaling, and a proteasome inhibitor such as, for example MG132 (EMD, CA), is supplied to a basic defined medium to support differentiation of adult pancreatic-derived stromal cells into a β-cell lineage. In one aspect, the cells are cultured in an induction media composed of DMEM (low glucose, 5.5 mM) containing 10 micromolar MG-132 for about one to about two days, followed by additional incubation for about three to about seven days in an induction media supplemented with 1X B27 (Gibco, CA) and 1X N2 (Gibco, CA) and further supplemented with Cyclopamine (10 µM; EMD, CA), bFGF (20 ng/ml; R&D Systems, MN), Activin A (20 nM; R&D Systems, MN) or FGF5 (20 ng/ml; R&D Systems, MN) for an additional five days.

In another aspect of the present invention, a combination of growth factors and chemical agents, including a proteasome inhibitor such as, for example MG132 (EMD, CA), is supplied to a basic defined medium to support differentiation of adult pancreatic-derived stromal cells into a β-cell lineage. In one aspect, the cells are cultured in induction media composed of DMEM (low glucose, 5.5 mM) containing a range of MG-132 concentrations from one to ten micromolar for about one to about two days, followed by additional incubation for about one to about two days with a histone deacetylase inhibitor such as, for example, Trichostatin A (Sigma, MO). Following the removal of these inhibitors, the cells are cultured in an induction media supplemented with factors appropriate for use in the induction medium which may include, for example, nicotinamide, members of TGF-β family, including TGF-β1, 2, and 3, bone morphogenic proteins (BMP-2, -4, 6, -7, -11, -12, and -13), serum albumin, fibroblast growth factor family, platelet-derived growth factor-AA, and -BB, platelet rich plasma, insulin growth factor (IGF-I, II) growth differentiation factor (GDF-5, -6, -8, -10, 11), glucagon like peptide-I and II (GLP-I and II), GLP-1 and GLP-2 mimetobody, Exendin-4, retinoic acid, parathyroid hormone, insulin, progesterone, aprotinin, hydrocortisone, ethanolamine, beta mercaptoethanol, epidermal growth factor (EGF), gastrin I and II, copper chelators such as triethylene pentamine, TGF-α, forskolin, Na-Butyrate, activin, betacellulin, ITS, hepatocyte growth factor (HGF), keratinocyte growth factor (KGF), bovine pituitary extract, islet neogenesis-associated protein (INGAP), proteasome inhibitors, histone deacetylase inhibitors, notch pathway inhibitors, sonic hedgehog inhibitors, or combinations thereof for an additional period of time to achieve the desired degree of differentiation to a β-cell lineage.

The combination and concentrations of growth factors, the length of culture, and other culture conditions can be optimized by those skilled in the art to achieve effective differentiation by, e.g., monitoring the percentage of cells that have differentiated into cells characteristic of the β-cell lineage. The one or more growth factors may be added in an amount sufficient to induce the differentiation of the pancreatic stromal cells of the present invention into cells bearing markers of a β-cell lineage over a time period of about one to four weeks.

Alternatively, the adult pancreatic-derived stromal cells of the present invention may be utilized in an *in vitro* assay to test for agents that are capable of inducing cells to differentiate into a β-cell lineage. Cells may be contacted with one or more than one agent and changes in gene expression monitored over time. An example of the method that may be employed is disclosed in **Example 9** below.

### Therapeutic Use of The Cells of The Present Invention.

It was found that the adult pancreatic-derived stromal cells isolated and expanded according to the methods of this invention maintain the ability to differentiate into cells of a pancreatic β-cell lineage. The cells of the present invention can be utilized for the treatment of Type 1 and 2 diabetes.

In one aspect, the cells of the present invention are used in treating a mammal suffering from, or at risk of developing Type1 diabetes. This involves culturing isolated adult pancreatic-derived stromal cells, expanding the isolated population of cells, and differentiating the cultured cells *in vitro* into a β-cell lineage. The differentiated cells can be used therapeutically by implantation either directly or in a pharmaceutical carrier into the mammal, although this step does not form part of the invention. If appropriate, pharmaceutical agents or bioactives that facilitate the survival and function of the transplanted cells can further be used to treat the mammal. These agents may include, for example, insulin, members of the TGF-β family, including TGF-β1, 2, and 3, bone morphogenic proteins (BMP-2, -3, -4, -5, -6, -7, -11, -12, and 13), fibroblast growth factors-1 and -2, platelet-derived growth factor-AA, and -BB, platelet rich plasma, insulin growth factor (IGF-I, II) growth differentiation factor (GDF-5, -6, -8, -10, -15), vascular endothelial cell-derived growth factor (VEGF), pleiotrophin, endothelin, among others. Other pharmaceutical compounds can include, for example, nicotinamide, glucagon like peptide-I (GLP-1) and II, GLP-1 and 2 mimetibody, Exendin-4, retinoic acid, parathyroid hormone, MAPK inhibitors, such as, for example, compounds disclosed in U.S. Published Application 2004/0209901 and U.S. Published Application 2004/0132729.

In yet another aspect, this invention provides cells for use in treating a mammal suffering from, or at risk of developing Type 2 diabetes. This involves culturing isolated adult pancreatic-derived stromal cells according to the present invention, expanding the isolated population of cells, and differentiating the cultured cells *in vitro* into a β-cell lineage. The differentiated cells can be used therapeutically by implantation either directly or in a pharmaceutical carrier into the mammal, although this step does not form part of the invention.

In yet another embodiment, the adult pancreatic-derived stromal cells of the present invention can be used therapeutically by transplantation with mature islets of the same or different animal species to enhance the survival of the stromal cells or to induce further differentiation of the stromal cells into a β-cell lineage.

The source of pancreatic tissue from which the stromal cells of the present invention are isolated can be autologous in relation to the mammal undergoing the therapeutic treatment. Alternatively, the source may be allogeneic, or xenogeneic. Cells for use in treatment of a mammal can also be genetically modified to enhance proliferation and/or differentiation or prevent or lessen the risk of immune rejection. Alternatively, the adult pancreatic-derived stromal cells obtained in accordance with the present invention can be used to modulate the recipient's immune response, prior to transplantation of differentiated cells prepared in accordance with the present invention. See, for example, U.S. Patent 6,328,960, U.S. Patent 6,281,012.

The adult pancreatic-derived stromal cells of the present invention can be fully differentiated into an insulin-producing cell, prior to use in therapy by transplantation into a recipient. In a specific embodiment, the adult pancreatic-derived stromal cells of the present invention are fully differentiated into β-cells, prior to use in therapy by transplantation into a recipient. Alternatively, the adult pancreatic-derived stromal cells of the present invention can be used in therapy by transplantation into a recipient in an undifferentiated or partially differentiated state. Further differentiation may take place in the recipient.

The cells, undifferentiated or otherwise, can be used as dispersed cells or formed into clusters that can be infused into the hepatic portal vein. Alternatively, the cells can be provided in biocompatible degradable polymeric supports, porous non-degradable devices or encapsulated to protect from host immune response. The cells can be used in therapy by implantation into an appropriate site in a recipient. The implantation sites include, for example, the liver, natural pancreas, renal subcapsular space, omentum, peritoneum, subserosal space or a subcutaneous pocket.

To enhance further differentiation, survival or activity of implanted cells, additional factors, such as growth factors, antioxidants or anti-inflammatory agents, can be used in therapy by administering them before, simultaneously with, or after the cells. In certain embodiments, growth factors are utilized to differentiate the administered cells *in vivo.* These factors can be secreted by endogenous cells and exposed to the administered stromal cells *in situ.* Implanted stromal cells can be induced to differentiate by any combination of endogenous and exogenously administered growth factors known in the art.

The amount of cells used in therapy by implantation depends on a number of factors including the patient's condition and response to the therapy, and can be determined by one skilled in the art.

In one aspect, this invention provides cells for use in treating a mammal suffering from, or at risk of developing diabetes. This includes culturing isolated adult pancreatic derived stromal cells according to the present invention, expanding the isolated population of cells, differentiating *in vitro* the cultured stromal cells into a β-cell lineage, and incorporating the cells into a three-dimensional support. The cells can be maintained *in vitro* on this support prior to use in therapy by implantation into the mammal. Alternatively, the support containing the cells can be used in therapy directly without additional *in vitro* culturing. The support can optionally be incorporated with at least one pharmaceutical agent that facilitates the survival, differentiation and function of the transplanted cells.

Support materials suitable for use for purposes of the present invention include tissue templates, conduits, barriers, and reservoirs useful for tissue repair. In particular, synthetic and natural materials in the form of foams, sponges, gels, hydrogels, textiles, and nonwoven structures, which have been used *in vitro* and *in vivo* to reconstruct or regenerate biological tissue, as well as to deliver chemotactic agents for inducing tissue growth, are suitable for use in practicing the methods of the present invention. See, e.g., the materials disclosed in U.S. Patent 5,770,417, U.S. Patent 6,022,743, U.S. Patent 5,567,612, U.S. Patent 5,759,830, U.S. Patent 6,626,950, U.S. Patent 6,534,084, U.S. Patent 6,306,424, U.S. Patent 6,365,149, U.S. Patent 6,599,323, U.S. Patent 6,656,488, and U.S. Patent 6,333,029. Exemplary polymers suitable for use in the present invention are disclosed in U.S. Published Application 2004/0062753 A1 and U.S. Patent 4,557,264.

To form a support incorporated with a pharmaceutical agent, the pharmaceutical agent can be mixed with the polymer solution prior to forming the support. Alternatively, a pharmaceutical agent could be coated onto a fabricated support, preferably in the presence of a pharmaceutical carrier. The pharmaceutical agent may be present as a liquid, a finely divided solid, or any other appropriate physical form. Alternatively, excipients may be added to the support to alter the release rate of the pharmaceutical agent. In an alternate embodiment, the support is incorporated with at least one pharmaceutical compound that is an anti-inflammatory compound, such as, for example compounds disclosed in U.S. Patent 6,509,369.

In one embodiment, the support is incorporated with at least one pharmaceutical compound that is an anti-apoptotic compound, such as, for example, compounds disclosed in U.S. Patent 6,793,945.

In another embodiment, the support is incorporated with at least one pharmaceutical compound that is an inhibitor of fibrosis, such as, for example, compounds disclosed in U.S. Patent 6,331,298.

In a further embodiment, the support is incorporated with at least one pharmaceutical compound that is capable of enhancing angiogenesis, such as, for example, compounds disclosed in U.S. Published Application 2004/0220393 and U.S. Published Application 2004/0209901.

In still another embodiment, the support is incorporated with at least one pharmaceutical compound that is an immunosuppressive compound, such as, for example, compounds disclosed in U.S. Published Application 2004/0171623.

In a further embodiment, the support is incorporated with at least one pharmaceutical compound that is a growth factor, such as, for example, members of the TGF-β family, including TGF-β1, 2, and 3, bone morphogenic proteins (BMP-2, -3, -4, -5, -6, -7, - 11,-12, and -13), fibroblast growth factors-1 and -2, platelet-derived growth factor-AA, and -BB, platelet rich plasma, insulin growth factor (IGF-I, II) growth differentiation factor (GDF-5, -6, -8, -10, -15), vascular endothelial cell-derived growth factor (VEGF), pleiotrophin, endothelin, among others. Other pharmaceutical compounds can include, for example, nicotinamide, hypoxia inducible factor 1-alpha, glucagon like peptide-I and II (GLP-1, -2), GLP-1 and GLP-2 mimetibody, , Exendin-4, retinoic acid, parathyroid hormone, tenascin-C, tropoelastin, thrombin-derived peptides, cathelicidins, defensins, laminin, biological peptides containing cell- and heparin-binding domains of adhesive extracellular matrix proteins such as fibronectin and vitronectin, MAPK inhibitors, such as, for example, compounds disclosed in U.S. Published Application 2004/0209901 and U.S. Published Application 2004/0132729.

The incorporation of the cells of the present invention into a scaffold can be achieved by the simple depositing of cells onto the scaffold. Cells can enter into the scaffold by simple diffusion (J. Pediatr. Surg. 23 (1 Pt 2): 3-9 (1988)). Several other approaches have been developed to enhance the efficiency of cell seeding. For example, spinner flasks have been used in seeding of chondrocytes onto polyglycolic acid scaffolds (Biotechnol. Prog. 14(2): 193-202 (1998)). Another approach for seeding cells is the use of centrifugation, which yields minimum stress to the seeded cells and enhances seeding efficiency. For example, Yang *et al.* developed a cell seeding method (J. Biomed. Mater. Res. 55(3): 379-86 (2001)), referred to as Centrifugational Cell Immobilization (CCI).

The present invention is further illustrated, but not limited by, the following examples.

### Example 1

### The Establishment of Human Adult Pancreatic-Derived Stromal Cell Lines

*Pancreas Preparation -* Human pancreata not suitable for clinical transplantation were obtained from The National Disease Research Interchange (Philadelphia, PA), following appropriate consent for research use. The pancreas was transferred with organ preservation solution to a stainless steel pan on ice and trimmed of all extraneous tissue. The pancreatic duct was cannulated with an 18-gauge catheter and the pancreas was injected with an enzyme solution, which contained the LIBERASE HI™ enzyme (Roche - 0.5 mg/ml) and DNase I (0.2 mg/ml), dissolved in Dulbecco's Phosphate Buffered Saline (DPBS).

*Rapid Mechanical Dissociation Followed by Enzymatic Digestion -* The enzyme infused pancreata were homogenized in a tissue processor, pulsed 3 to 5 times for 3 to 5 seconds/pulse, and the dissociated tissue were transferred to two 500 ml trypsinizing flasks (Bellco) containing magnetic stir bars. Thereafter, 50 to 100 ml of the enzyme solution was added to each flask. The flasks were placed in a 37°C water bath on submersible stir plates and allowed to incubate with an intermediate stir rate for 10 minutes. The stirring was stopped and the fine digested tissue was removed from the flask and transferred into 250 ml tube containing DPBS, 5% Fetal Bovine Serum (FBS) and 0.1 mg/ml DNase I (DPBS+) at 4°C to quench the digestion process. The flasks were replenished with 50 to 100 ml of the enzyme solution and returned to the water bath and the stirring was re-initiated for an additional ten minutes. Again, the flasks were removed and the fine digest was collected and transferred to the 250 ml tubes on ice. This process was repeated for additional 3-5 times until the pancreas was completely digested.

*Gradual Mechanical Dissociation with Simultaneous Enzyme Digestion -* The enzyme infused pancreata were processed according to methods as described in Diabetes 37:413-420 (1988). Briefly, the pancreata were cleaned of extraneous tissue and injected with the enzyme solution as described above. The pancreata were then placed into a Ricordi Chamber with beads and covered with a screen with a mesh size of 400 to 600 µm to retain larger clusters of tissue. The chamber was covered and the enzyme solution was circulated through the chamber at approximately 37°C and the chamber was shaken to allow beads to disrupt pancreatic tissue while the enzyme digested the pancreas. Once adequate dissociation and digestion was achieved, the digestion was terminated and the tissue was collected.

*Tissue Separation -* The collected tissue was centrifuged at 150 x g for 5 minutes at 4°C. The supernatant was aspirated and the tissue was washed two additional times in DPBS+. Following the final wash, the tissue was applied to a discontinuous gradient for purification. The digested tissue was suspended in polysucrose (Mediatech, VA) with a density of 1.108 g/ml at a ratio of 1 to 2 ml tissue pellet per 10 ml of polysucrose solution. The tissue suspension was then transferred to round-bottom polycarbonate centrifuge tubes and polysucrose solutions with densities of 1.096 and 1.037 were carefully applied to the tubes. A final layer of DMEM completed the discontinuous purification gradient. The gradient tubes were centrifuged at 2000 rpm for 20 minutes at 4°C with no brake applied. Following centrifugation, the tissue was collected from each interface (three interfaces) and washed several times in DPBS+ as described above and collected in a 50 ml test tube.

*Further Cell Cluster Dissociation -* Optionally, one can further dissociate large cell clusters obtained using the above protocol into smaller clusters or single cell suspensions. After the final wash, the tissue from each fraction was suspended in 10 ml 1X trypsin/EDTA solution containing 200U/ml DNase I. The tubes were placed in the water bath and repeatedly aspirated and discharged from a 10 ml serological pipette for 5 to 6 minutes until a near single cell suspension is achieved. The digestion was quenched with the addition of 4°C DPBS+ and the tubes centrifuged at 800 rpm for 5 minutes. The cell suspensions were washed with DPBS+ and cultured as described below.

*Pancreatic Cell Culture -* Following the final wash, the cells from each interface were resuspended in DMEM, 2% FBS, 100 U/µg penicillin/streptomycin, ITS, 2 mM L-Glutamine, 0.0165 mM ZnSO₄ (Sigma), and 0.38 µM 2-mercaptoethanol (Invitrogen, CA) (hereinafter "the selection media"). Six ml of the cell suspension was seeded in T-25 tissue culture flasks and 12 ml of the cell suspension was seeded into T-75 flasks. The flasks were placed in 37°C incubators with 5% CO₂. Following two to about four weeks culture, a complete media change was performed and adherent cells were returned to culture in DMEM (2750 mg/l D-glucose, 862 mg/l glutamine) (Gibco, CA) with 5% FBS (HyClone, UT), 1% P/S, 0.0165 mM ZnSO₄ (hereinafter "the growth media") and allowed to reach near confluence (this stage is referred to as "passage 0" or "P0"), at which point they were passaged. Subsequent culturing of the cells was at 5000 cell/cm² in the growth media. Cultures were passaged every seven to ten days at approximately 70 to 90% confluency. **Figure 2** depicts the typical morphology of the adult pancreatic-derived stromal cells of the present invention.

### Example 2

### Population Doubling Time

Passage 10 and 12 adult pancreatic-derived stromal cells isolated and expanded according to **Example 1** above were seeded at 10000 cells/well of a 24-well tissue culture plate (Corning, MA) in the growth media. At various time points, cells were removed from three wells of the plate using TRYPLE™ Express (Invitrogen, CA) and counted using a Guava PCA-96 cell analysis system and the VIACOUNT® reagent (Guava, CA). **Figure 3** depicts the growth curve of passage 10 cells cultured under normoxic conditions. The linear phase of the log plot was used to estimate the population doubling time of the cells. Population doubling time of passage 10 and 12 cells was 36 hrs and 38 hrs, respectively.

### Example 3

### Expansion Potential of Adult Pancreatic-Derived Stromal Cells

Adult pancreatic-derived stromal cells isolated according to **Example 1** were cultured in a 75cm² tissue culture treated flask (Corning, MA) under normoxic conditions (5% CO₂, 20% O₂, and 75% N₂) for 3 wks in the selection media. The cultures were then switched to growth media and fed two to three times per week. The cells were allowed to reach near confluence, at which point they were passaged. Cultures were passaged every 7-14 days at approximately 70 to 95% confluency for the first 9 passages during which time the cell population doubling time was greater than 100 hrs. Following the tenth passage the cell growth rate increased and the population doubling time was reduced to approximately 40 hrs with the cells passaged every 4 to 6 days. Cells were harvested from flasks at each passage using TRYPLE™ Express (Invitrogen, CA) and counted using a Guava PCA-96 cell analysis system and the VIACOUNT® reagent (Guava, CA). The cells continue to proliferate in expansion media culture for 132 days, currently reaching 40 population doublings and a projected 10¹² fold increase in cell numbers. (See **Figure 6**)

### Example 4

### Expansion Potential of a Number of Adult Pancreatic-Derived Stromal Cell Lines

Adult pancreatic-derived stromal cells isolated according to **Example 1** were either cultured under hypoxic conditions (5% CO₂, 3% O₂, and 92% N₂) or normoxic conditions (5% CO₂, 20% O₂, and 75% N₂) for two to four weeks in the selection media. The cultures were then switched to the growth media and fed two to three times per week. After the initial culture period, adherent cells were observed in plates cultured under hypoxic and normoxic conditions. Furthermore, following the initial two to four weeks of culturing, there were very few remaining islet-like or ductal structures in the plates.

The *in vitro* expansion potential of the adult pancreatic-derived stromal cell lines, isolated from a number of donor pancreata was tested, using a variety of media. Cells from fractions 3, 4 and 5 were cultured under normoxic conditions on tissue culture treated flasks, in media comprising DMEM, 5% FBS, 25mM HEPES, 0.0165 mM ZnSO₄,. Parallel populations of cells were cultured in CMRL1066, 10% FBS, 25mM HEPES, or DMEM, 10% FBS, 25 mM HEPES, all cultures were supplemented with antibiotics. Cultures were allowed to reach confluence before passage. **Table VI** describes the population doubling time (PDT), the total number of population doublings (PD) and the projected cell number, of the cell lines cultures under the conditions tested.

### Example 5

### Selection of CD105 Positive Cells

At about 70% confluency, P0 adult pancreatic-derived cells isolated according to **Example 1** were released using the TRYPLE™ Express (Invitrogen, CA) solution, followed by rinsing with the growth media (DMEM, 5% FBS, 0.0165 mM ZnSO₄ and 1% P/S). The cell suspension was centrifuged for 5 mins at 1400 RPM, and the supernatant was discarded. The resulting cell pellet was resuspended in phosphate buffered saline (PBS), supplemented with 0.5% bovine serum albumin (BSA) and 2 mM EDTA. The cells were selected for a CD105 positive population following manufacturer's instructions (Miltenyi Biotech, CA). The isolation of CD105 positive fraction was confirmed by using flow cytometry and using mouse anti-human CD105PE-labeled antibody (Santa Cruz, CA). The positive fraction was cultured in growth media. The CD105 positive fraction consisted mainly of proliferating adult pancreatic-derived stromal cells.

### Example 6

### Fluorescence-Activated Cell Sorting (FACS) Analysis

Adhered cells were removed from passage 9-12 plates by five-minute incubation with the TRYPLE™ express solution (Gibco, CA). Released cells were resuspended in DMEM supplemented with 10% FBS and recovered by centrifugation, followed by washing and resuspending the cells in a staining buffer consisting of 2% BSA, 0.05% sodium azide (Sigma, MO) in PBS. If appropriate, the cells were Fc-receptor blocked using a 0.1% γ-globulin (Sigma) solution for 15 mins. Aliquots (approximately 10⁵ cells) were incubated with either phycoerythirin (PE) or allophycocyanin (APC) conjugated monoclonal antibodies (5 µl antibody per 10⁶ cells), as indicated in **Table II-A,** or with an unconjugated primary antibody. Controls included appropriate isotype matched antibodies, non-stained cells, and cells only stained with secondary conjugated antibody. All incubations with antibodies were performed for 30 mins at 4°C, after which the cells were washed with the staining buffer. Samples that were stained with unconjugated primary antibodies were incubated for additional 30 mins at 4°C with secondary conjugated PE or -APC labeled antibodies. See Table II-B for a list of secondary antibodies used. Washed cells were pelleted and resuspended in the staining buffer and the cell surface molecules were identified by using a FACS Array (BD Biosciences) by collecting at least 10,000 events.

*For intracellular staining, cells were first fixed for 10 mins with 4% paraformaldheyde, followed by two rinses in the staining buffer, centrifugation, of cells and resuspension of the cells in a permeabilization buffer containing 0.5% Triton-X (Sigma) in PBS for 5 mins at room temperature (RT). The permeabilized cells were rinsed twice with a rinsing buffer, centrifuged, and resuspended in the staining buffer and incubated with an appropriate conjugated antibody (5 µl antibody per 10⁶ cells), as indicated in **Table II-C** for 30 mins at 4°C. Samples that were stained with unconjugated primary antibodies were incubated for additional 30 mins at 4°C with secondary conjugated PE or -APC labeled antibodies (**Table II B**)**.** Washed cells were pelleted and resuspended in the staining buffer and the internal proteins were identified by using a FA CSArray (BD Biosciences) by collecting at least 10,000 events. The expression level of examined surface and internal markers is listed in Table III and **Table IV. Figure 4** depicts a sample FACSprofile of passage 9 cells.*

### Example 7

### Immunostaining of Adult Pancreatic-Derived Stromal Cells

10,000 cells/cm² passage 10 adult pancreatic-derived stromal cells, cultured according to **Example 1**, were seeded into glass bottom 35 mm microwell dishes (Matek Corp, MA) in growth media. Following three days in culture, the cells were fixed for 10 mins with 4% paraformaldheyde, followed by two rinses in the PBS, and addition of a permeabilization buffer containing 0.5% Triton-X (Sigma) for 5 mins at room temperature (RT) followed by additional three rinses with PBS. The fixed and permeabilized cells were blocked with either 1% bovine serum albumin (BSA) or 4% sera from the species where the secondary antibody was raised in (Goat, donkey, or rabbit). Primary and secondary antibodies used are listed in **Table V A-B.** Control samples included reactions with the primary antibody omitted or where the primary antibody was replaced with corresponding immunoglobulins at the same concentration as the primary antibodies. Stained samples were rinsed with a PROLONG® antifade reagent (Invitrogen, CA) containing diamidino-2-phenylindole, dihydrochloride (DAPI) to counter stain the nucleus. Images were acquired using a Nikon Confocal Eclipse C-1 inverted microscope (Nikon, Japan) and a 60X objective. During the expansion phase, a majority of the cells stained positive for smooth-muscle actin, Nestin, vimentin, beta III tubulin, and GATA4 **(Figure 5),** a minority of cells (less than 5%) stained positive for GFAP, and none of the cells stained positive for CK 7 or CK 19.

### Example 8

### PCR Analysis Of Adult Pancreatic-Derived Stromal Cells

RNA was extracted from passage 9 cells cultured in the growth media. RNA collected from human pancreas was used as positive control; and bone marrow derived mesenchymal cells (Cambrex, MD) was used as negative controls for the expression of key genes involved in pancreatic development.

*RNA extraction, purification, and cDNA synthesis.* RNA samples were purified through its binding to a silica-gel membrane (Rneasy Mini Kit, Qiagen, CA) in the presence of an ethanol-containing, high-salt buffer; while contaminants were washed away. The RNA was further purified while bound to the column by treatment with DNase I (Qiagen, CA) for 15 min. High-quality RNA was then eluted in water. Yield and purity were assessed by A260 and A280 readings on the spectrophotometer. cDNA copies were made from purified RNA using an ABI (ABI, CA) high capacity cDNA archive kit.

*Real-time PCR amplification and quantitative analysis.* Unless otherwise stated, all reagents were purchased from Applied Biosystems. Real-time PCR reactions were performed using the ABI PRISM® 7000 Sequence Detection System. TAQMAN® UNIVERSAL PCR MASTER MIX® (ABI, CA) was used with 20 ng of reverse transcribed RNA in a total reaction volume of 20 µl. Each cDNA sample was run in duplicate to correct for pipetting errors. Primers and FAM-labeled TAQMAN®probes were used at concentrations of 200 nM. The level of expression of each target gene was normalized using the pre-developed Applied Biosystem's 18S ribosomal RNA or human glyceraldehydes-3-phosphate dehydrogenase (GAPDH) endogenous control kit. Primers and probes were either designed using ABI PRISM PRIMER EXPRESS™ software or used pre-developed ABI gene analysis kit. For each gene, either one of the primers or the probe were designed to be exon-boundary spanning. This eliminated the possibility of the primers/probe binding to any genomic DNA present. The primer and probe sets are listed as following Nkx2.2 (Hs00159616), Pdx-1 (Hs00426216), Nkx6.1 (Hs00232355), Ngn3 (Hs00360700), Pax4 (Hs00173014), Pax6 (Hs00240871), Insulin (Hs00355773), Glu2 (Hs00165775), glucagon (Hs00174967), Isl-1 (Hs00158126), somatostatin (Hs00174949), FoxA2 (Hs00232764), HlxB9 (Hs00232128), GATA-4 (Hs00171403), GFAP (Hs00157674), MAP2 (Hs00159041), Olig2 (Hs00377820) and Oct-4 (CGACCATCTGCCGCTTTGAG (SEQ ID NO: 1) and CCCCCTGTCCCCCA TTCCTA (SEQ ID NO: 2)) Rex-1 (CAGATCCTAAACAGCTCGCAGAAT (SEQ ID NO: 3) and GCGTACGCAAATTAAACTCCAGA(SEQ ID NO: 4)). After an initial 50°C for 2 min, and 95°C for 10 min, samples were cycled 40 times in two stages - a denaturation step at 95°C for 15 sec followed by an annealing/extension step at 60°C for 1 min. Data analysis was carried out using GENEAMP®7000 Sequence Detection System software. For each primer/probe set, a Cₜ value was determined as the cycle number at which the fluorescence intensity reached a specific value in the middle of the exponential region of amplification. Relative gene expression levels were calculated using the comparative Cₜ method. Briefly, for each cDNA sample, the endogenous control Cₜ value was subtracted from the gene of interest Cₜ to give the delta Cₜ value (ΔCₜ). The normalized amount of target was calculated as 2^{-ΔCt}, assuming amplification to be 100% efficiency. Final data were expressed relative to a calibrator sample. The comparative Cₜ method is only valid if target and endogenous control amplification efficiencies are approximately equal. Preliminary validation experiments were therefore performed for each primer/probe set by amplifying serially diluted cDNA samples and determining the ΔCₜ values. These ΔCₜ values remain constant across the range of dilutions if amplification efficiencies are equal.

PCR data obtained from adult pancreatic-derived stromal cells cultured in either DMEM + 2% FBS, or DMEM + 10% FBS, or CMRL + 10% FBS showed that the cells expressed PDX-1, insulin and HNF-3β initially, following isolation. However, the expression of these genes rapidly decreased, such that by passage 9, expression of these genes was undetectable by real-time PCR (Figures 7-19). Expression of these genes remained undetectable for up to passage 30. These data suggest that the adult pancreatic-derived stromal cells isolated as described above remained fully undifferentiated cells while being cultured under growth conditions.

### Example 9

### Differentiation Protocols

1x10⁴ cells/cm² of passage 13 adult pancreatic-derived stromal cells were seeded in a 24 well plate (Corning, CA) and cultured under standard conditions at 37°C in a 5% CO₂ incubator in the growth media until 100% confluent, after which they were cultured for 24 hours in DMEM (1000mg/L D-glucose) with 10 µM of MG132 (EMD, CA) for 24 hours. Cells were washed once with PBS and cultured in DMEM (1000 mg/l D-glucose) supplemented with 1X B27 (Gibco, CA) and 1X N2 (Gibco, CA) and further supplemented with Cyclopamine (10 µM; EMD, CA), bFGF (20 ng/ml; R&D Systems, MN), Activin A (20 nM; R&D Systems, MN) or FGF5 (20 ng/ml; R&D Systems, MN) for additional 5 days. RNA was collected from cells and analyzed for the expression of PDX-1 and insulin by methods outlined in **Example 7.** These culture conditions induced the expression of PDX-1, but not insulin.

In a separate experiment, adult pancreatic-derived stromal cells from passage 12 and passage 19 from fraction 3 were seeded at 10⁵ cells/well into 6-well tissue culture plates (BD Falcon, NJ) and allowed to reach confluence whereupon 1 or 10 µM of the gamma secretase inhibitor InSolution™ MG-132 (Calbiochem, CA) was added for 1 day. RNA was collected from cells and analyzed for the expression of HNF3β, PDX-1, Nkx6.1 and insulin. The treatment with InSolution™ MG-132 alone did not lead to the expression of key β-cell lineage genes such as HNF3β, PDX-1, Nkx6.1 or insulin. Following InSolution™ MG-132 treatment for 1 day, the cells were treated with 1.25 or 2.5 µM of the histone deacetylase inhibitor Trichostatin A (Sigma, MO) for one day. RNA was collected from cells and analyzed for the expression of HNF3β, PDX-1, Nkx6.1 and insulin. The combination of the two agents resulted in the expression of HNF3β, PDX-1, Nkx6.1, and insulin (**Table VII and Figure 20**).

In a separate experiment, adult pancreatic-derived stromal cells from passage 18 from fraction 3 were seeded at 10⁵ cells/well into 6-well tissue culture plates (BD Falcon, NJ) and allowed to reach confluence whereupon 10 µM of the gamma secretase inhibitor [(2R, 4R, 5S)-2-Benzyl-5 (Boc-amino)-4-hydroxy-6-phenyl-hexanoyl]-Leu-Phe-NH₂ (Sigma, MO, hereafter referred to as B5306) was added for 1, 3, 7, 10, and 14 days. RNA was collected from cells and analyzed for the expression of HNF3β, PDX-1, Nkx6.1 and insulin. The treatment with B5306 alone did not lead to the expression of β-cell lineage genes such as HNF3β, PDX-1, Nkx6.1 or insulin. Following B5306 treatment for 1,7, or 14 days the cells were treated with 1.25 or 2.5 µM of the histone deacetylase inhibitor Trichostatin A (Sigma, MO) for one day. RNA was collected from cells and analyzed for the expression of HNF3β, PDX-1, Nkx6.1 and insulin. The combination of the two agents resulted in the expression of HNF3β, PDX-1, Nkx6.1, but not insulin (**Table VIII**).

In a separate experiment, adult pancreatic-derived stromal cells from passage 5 were seeded into 6-well tissue culture plates at 10⁵ cells/well and incubated with the induction media listed in **Table IX** for two weeks with a complete media change twice a week. Following two weeks incubation, RNA was collected from representative wells and analyzed for the expression of pancreatic genes. The cells were found to be positive for glucagon, Nkx6.1, Pax6, and somatostatin but not insulin, PDX-1, or HNF3β (**see Table X** - differentiation column). In a parallel experiment, the effects of cell aggregation on pancreatic gene expression were examined. Following the two weeks incubation with the various induction media, the induction media was removed and the adult pancreatic-derived stromal cells were treated with 0.05% trypsin/EDTA (Invitrogen, CA) for two minutes whereupon it was removed. Serum free media consisting of CMRL 1066 (Invitrogen, CA), 1% BSA (Sigma, MO), and ITS (Invitrogen, CA) was then added to each of the remaining wells. The following day the cells had formed islet-like aggregates and RNA was collected and analyzed for the expression of pancreatic genes. The cells were found to be positive for insulin and HNF3β by methods outlined in **Example 7** (**see Table X -** aggregation column).

In a separate experiment, adult pancreatic-derived stromal cells from passage 13 were seeded into 6-well tissue culture plates at 10⁵ cells/well and incubated with the induction media listed in **Table IX** for two weeks with a complete media change twice a week. Following two weeks incubation, RNA was collected from representative wells and analyzed for the expression of pancreatic genes. The cells were found to be positive for glucagon, Nkx6.1, Pax6, and somatostatin but not insulin, PDX-1, or HNF3β (**see Table X -** differentiation column). In a parallel experiment, the effects of cell aggregation on pancreatic gene expression were examined. Following the two weeks incubation with the various induction media, the induction media was removed and the cells were treated with 0.05% trypsin/EDTA (Invitrogen, CA) for two minutes whereupon it was removed. Serum free media consisting of CMRL 1066 (Invitrogen, CA), 1% BSA (Sigma, MO), and ITS (Invitrogen, CA) was then added to each of the remaining wells. The following day the cells had formed islet-like aggregates and RNA was collected and analyzed for the expression of pancreatic genes. The cells were found to be positive for insulin and HNF3β by methods outlined in **Example 7** (**see Table X -** aggregation column).

Taken together, these data suggest that the adult pancreatic-derived stomal cells of the present invention are capable of differentiating towards cells of the β-cell lineage..

### Example 10

### Cryopreservation of Adult Pancreatic-Derived Stromal Cells

Adult-pancreatic-derived stromal cells isolated according to the methods described in **Example 1** were collected at the desired passage number and are resuspended in 1 to 2 ml of 90% FBS (HyClone, UT) and 10% DMSO (Sigma, MO) at a concentration of 1 to 5 x 10⁶ cells/ml. The cell suspension was aliquoted into cryogenic vials (Corning, NY) and transferred to a Nalgene Cryo 1 °C Freezing Container and placed into a - 80°C freezer for a minimum of 4 hrs. The vials were removed from -80°C and transferred to the vapor phase of a liquid nitrogen storage tank until needed. The cells were thawed by removing the cryogenic vials from the vapor phase of a liquid nitrogen storage tank and transferring them immediately to a 37° water bath and for 1 to 2 minutes or until only a small ice crystal remained. The cells were washed with culture media and placed into 37°C culture as described in the above examples. The cells were then passaged as necessary. The data shown in **Figure 21** demonstrated that the cryopreservation had little effect on the expansion potential and growth rate.

### Example 11

### Effects of Nutrient Deprivation on Adult Pancreatic-Derived Stromal Cell Growth

Adult pancreatic-derived stromal cells isolated according to the methods described in **Example 1** were subjected to different culture conditions and evaluated for the effects of these conditions on subsequent cell growth dynamics. Cells isolated from islet depleted/acinar rich fraction, H8F5 were culture in DMEM, 5.5 mM glucose (Invitrogen, CA), 10% FBS (HyClone, UT), 2mM Glutamax, 25 mM HEPES and 1X AB/AM (Invitrogen, CA). In one culture, the cells were fed with fresh media every 2 to 3 days and by day 8 cells had reached confluence. The cells were passaged once a week thereafter upon reaching confluence in the culture flask. In the second culture, cells were allowed to grow for 7 days prior to media replacement that resulted in lower degree of attachment and expansion as compared to the cells re-fed more frequently. This culture did not reach confluence until 12 days after seeding the flask at which point the cells were passaged. Thereafter, both cells were fed every 2 to 3 days and passaged upon reaching confluence. The cells were counted at each passage and the projected number of cells was calculated from the number of cells originally seeded in the flask and those recovered.

Initially the cells proliferated at the same pace as is seen in **Figure 22.** However following the first few passages, cells that had been deprived of media replacement in their early culture demonstrated an increase in growth rate as compared to those cells that underwent a complete media change every 2 to 3 days after establishing the culture. As shown in **Figure 22,** the population doubling time was calculated as 84.2 hrs for this first culture and 58.5 hrs for the cells starved from media for the first 7 days following their establishment in culture. These results suggest that nutrient deprivation is a means to selectively establish adult pancreatic-derived stromal cells with an increased ability to expand as compare to conventional cell culture techniques.

### Example 12

### Cytogeneic Analysis of a Representative Population of Adult Pancreatic-Derived Stromal Cells of the Present Invention.

Adult pancreatic-derived stromal cells from passage 13 underwent cytogenetic analysis conducted in compliance with Food and Drug Administration Good Laboratory Practice Regulations set forth in Part 58 of Title 21, CFR. The cells were grown in monolayers in T-75 tissue culture flasks with DMEM, 5.5 mM glucose (Invitrogen, CA), 10% FBS (HyClone, UT), 2mM Glutamax, 25 mM HEPES and 1X AB/AM (Invitrogen, CA). When cultures were judged to have a suitable amount of mitotic cells, chromosome harvests were performed. The cells were treated with Colcemid (0.02 to 0.03 µg/ml) for 2 to 18 hours at 37°C. Thereafter, the cells were trypsinized, centrifuged for 6 to 7 minutes at 200 x g, and the supernatant removed.

The cells were resuspended in warm hypotonic solution at 37°C for 10 to 16 minutes and then centrifuged as described above. The cells were then fixed with Carnoy's fixative (3:1 methanol:glacial acetic acid) at room temperature for 36 to 50 minutes and washed with Carnoy's fixative twice, and then resuspended in freshly prepared fixative to produce an opalescent cell suspension. Drops of the final cell suspension were placed on clean slides and air-dried.

**Chromosome Count per 100 Metaphases.** The distribution of Chromosome numbers found in the 100 metaphases analyzed is shown in **Table XII.** The chromosome count ranged from 45 to 46 chromosomes per metaphase with a modal chromosome number of 46. Six polyploidy metaphases (0.6%) were recorded per 1000 cells analyzed.

**Chromosome Aberration.** The chromosome aberration data for the 100 metaphases analyzed are summarized in **Table XIII.** The cytogeneic data include the total number of cells analyzed for aberrations, the total number of aberrant cells, and the total number of aberrations. No chromosome aberrations were found in the 100 cells analyzed.

**G-Banded Chromosome Analysis/Karyotype.** Five karyotypes were prepared (Human Karyotype #1 through #5). Cytogeneic analysis shows that the cell line is of human origin (See **Figures 23 to 27**). Normal autosomes were present in all karyotypes as were the X and Y chromosomes. All unidentifiable chromosomes and rearrangements were classified as unidentifiable chromosomes (UC). Two of the five karyotypes analyzed contained one UC per karyotype.

### Example 13

### Micro Array Analysis of Adult Pancreatic-Derived Stromal Cells Derived from Fractions 3, 4, and 5 from a Human Pancreas.

Human pancreas H5 line was processed according to methods outlined in **Example 1.** Cells at passage 6-8 isolated from islet rich fraction (fraction 3), ductal rich fraction (fraction 4), and exocrine rich fraction (fraction 5) were used for gene expression profiling. Total RNA was isolated from human H5 line (fractions 3, 4, and 5) and human pancreas RNA (Ambion) using an RNeasy mini kit (Qiagen). The sample preparation, hybridization, and image analysis was performed according to the CodeLink™ System (GE Healthcare, Amersham Biosciences, NJ). Codelink™ Human Whole Genome arrays were used. It is comprised of approximately 55 000 30-mer probes designed to conserved exons across the transcripts of targeted genes. The chip contains ∼45000 unique Unigene IDs. Following normalization and a log transformation, data analysis was performed using OmniViz® software (MA) and GENESIFTER (VizXLabs, WA). The variance stabilizing transformation along with cross sample normalization was applied to the log transformed array dataset. The variability within each cell line and among the different cell lines was compared using the Pearson correlation coefficient. For each cell line, three biological and two technical replicates were used. For all the samples analyzed, the correlation coefficient within a cell line was higher as compared to those between the lines. Variance in gene expression profiles between the different cell types are depicted in **Figure 28.** Significant differences in gene expression between the cell types was evaluated using analysis of variance and an F-test with adjusted P-value of ≼ 0.05.

**Tables XIV-XVI** lists the genes that are differentially expressed at least 5-fold between the various cell lines.

### Example 14

### Micro Array Analysis of Adult Pancreatic-Derived Stromal Cells from Two Representative Donor Pancreata.

Two donor human pancreata were processed according to methods outlined in **Example 1.** Adult pancreatic-derived stromal cells at passage 9-11 isolated from islet rich fraction (fraction 3) were used for gene expression profiling. Total RNA was isolated from cells using an RNeasy mini kit (Qiagen). RNA was purified , cRNA was labeled and fragmented as per the manufacturers protocols (Affymetrix, CA). Labeled cRNA was hybridized to the U133 2.0 plus Affymetrix human gene chips, which was scanned using an Affymetrix GeneChip Analyzer. For each cell line, three biological and two technical replicates were used. Data was analyzed using OmniViz® software (MA) and GENESIFTER (VizXLabs, WA). The average correlation coefficient between the H8 replicates was 0.893 (0.892-0.895, n=3) and was 0.906 (0.903-0.909, n=3) for H9 replicates. Scatter plot comparing gene expression profile of H8F3 vs. H9 F3 cells is depicted in **Figure 29.**

**TABLE I. COMPARISON OF THE CELL OF THE PRESENT INVENTION WITH PANCREATIC STEM CELLS OF THE ART.**

| | **Cell 1** | **Cell 2** | **Cell 3** | **Cell 4** | **Cell 5** | **Cell 6** | **Cell 7** | **Cell 8** | **Cell 9** | **Undifferentiated Cell Present invention** |
|---|---|---|---|---|---|---|---|---|---|---|
| **Amylase** | | | | | | | | | | POS |
| **α1-antitrypsin** | | | | | | | | POS | | |
| **α3 integrin** | | | | | | | | | | POS |
| **α6 integrin** | | | | POS | | | | | | |
| **ABCG2** | | | | POS | | | | | | NEG |
| | | | | | | | | | | |
| **β6 integrin** | | | | POS | | | | | | |
| **βIII tubulin** | | | | | | | | | | POS |
| **c-Met** | | | | | | | | | | NEG |
| **C/EBP α** | | | | | | | | POS | | |
| **C/EBP β** | | | | | | | | POS | | |
| **CD1d** | | | | | | | | | | NEG |
| **CD9** | | | | | | | | | | POS |
| **CD13** | | | | | | | | | | POS |
| **CD34** | | | | NEG | | | | | | |
| **CD44** | | | | | | | | | | POS |
| **CD45** | | | | NEG | | | | | | NEG |
| **CD49b** | | | | | | | | | | POS |
| **CD49e** | | | | | | | | | | POS |
| **CD49f** | | | | | | | | | | NEG |
| **CD73** | | | | | | | | | | POS |
| **CD81** | | | | | | | | | | POS |
| **CD90** | | POS | | | | | | POS | | POS |
| **CD91** | | | | | | | | | | NEG |
| **CD95** | | | | | | | | | | POS |
| **CD105** | | POS | | | | | | | | POS |
| **CD117** | | | | POS | | | | | | NEG |
| **CD124** | | | | | | | | | | NEG |
| **CD133** | | | | NEG | | | | | | NEG |
| **CD138** | | | | | | | | | | NEG |
| **CD151** | | | | | | | | | | POS |
| **CD166** | | | | | | | | | | POS |
| **CD184** | | | | | | | | | | NEG |
| **CD221** | | | | | | | | | | NEG |
| **Cytokeratin 5** | | | | | | | | | | NEG |
| **Cytokeratin 6** | | | | | | | | | | NEG |
| **Cytokeratin 7** | | POS | | | | | | POS | | NEG |
| **Cytokeratin 8** | | | | | | | | POS | | NEG |
| **Cytokeratin 10** | | | | | | | | | | NEG |
| **Cytokeratin 13** | | | | | | | | | | NEG |
| **Cytokeratin 14** | | | | | | | | | | NEG |
| **Cytokeratin 15** | | | | | | | | | | NEG |
| **Cytokeratin 16** | | | | | | | | | | NEG |
| **Cytokeratin 18** | | | | | | | | POS | | NEG |
| **Cytokeratin 19** | | POS | | NEG | | | | POS | | NEG |
| **E-Cadherin** | | | | | | | | | | NEG |
| **EPCAM** | | | | | | | | | | NEG |
| **ErbB2** | POS | | | | | | | | | |
| **ErbB3** | POS | | | | | | | | | |
| **ErbB4** | POS | | | | | | | | | |
| **GATA4** | | | | | | | | | | POS |
| **GLP-1 R** | | | | POS | | | | | | NEG |
| **Glut-2** | | | | | | | | | | NEG |
| **Glucokinase** | | | | | | | | | | NEG |
| **Hes-1** | | | | POS | | | | | | |
| **HNF-1** | | | | | | | | POS | | NEG |
| **HNF3β** | | | | | | | | | | NEG |
| **HNF4α.** | | | | | | | | | | NEG |
| **HNF6** | | | | | | | | | | NEG |
| **Insulin** | | | | | | | | | | NEG |
| **Islet-1** | | | | | | | | | | POS |
| **IPF-1** | | | | | | POS | | | | |
| **Kir 6.2** | | | | POS | | | | | | |
| **latrophillin** | | | | POS | | | | | | |
| **MDR-1** | | | | POS | | | | | | |
| **MHC I** | | | | NEG | | | | | | |
| **MHC II** | | | | NEG | | | | | | |
| **MMP2** | | POS | | | | | | NEG | | |
| **Msx-2** | POS | | | | | | | | | |
| **NCAM** | | | | | | | | | POS | NEG |
| **Nestin** | | POS | POS | POS | | | | | | POS |
| **NeuroD** | | | | | | | | | | NEG |
| **Ngn-3** | | | POS | | | | | | | |
| **Nkx2.2** | | | | | | | | | | NEG |
| **Nkx6.1** | | | | | | | | | | NEG |
| **Oct 3** | | | | POS | | | | | | |
| **Oct 4** | | | | POS | | | | | | |
| **Par-2** | | | | | | | | | | NEG |
| **Pax4** | | | | | | | | | | NEG |
| **Pax6** | | | | | | | | | | NEG |
| **P4HA1** | | POS | | | | | | | | |
| **PDX-1** | | | POS | | | POS | | | | NEG |
| **PECAM** | | | | | | | | | | NEG |
| **pi-GST** | | | | | | | | POS | | |
| **Pref-1** | | | | | | POS | | | | |
| **PC 1/3** | | | | | | | | | | NEG |
| **smooth muscle actin** | | POS | | | | | | | | POS |
| **Snal1** | | POS | | | | | | | | |
| **Snal2** | | POS | | | | | | | | |
| **Somatostatin** | | | | | | | | | | NEG |
| **Sox-2** | | | POS | | | | | | | |
| **SSTR234** | | | | POS | | | | | | |
| **Sur-1** | | | | POS | | | | | | |
| **Thrombin** | | | | | | | | | | NEG |
| **Vimentin** | | POS | | | | | | | | POS |

**TABLE II A. ANTIBODIES TO SURFACE RECEPTORS**

| Antibody | Supplier | Isotype | Clone |
|---|---|---|---|
| A3 integrin | Santa Cruz Biotechnology (CA) | Mouse IgG1 | P1B5 |
| Alkaline phosphatase | R&D systems (MN) | Mouse IgG1 | B4-78 |
| ATP binding cassette transporter (ABCG2) | BD Pharmingen (CA) | Mouse IgG2b, Kappa | 5D3 |
| CD1d | BD Pharmingen (CA) | Mouse IgG1, kappa | M-T101 |
| CD9 | BD Pharmingen (CA) | Mouse IgG1, kappa | M-L13 |
| CD13 | Santa Cruz Biotechnology (CA) | Mouse IgG1 | WM-47 |
| CD49b | BD Pharmingen (CA) | Mouse IgG2A, kappa | 12F1-H6 |
| CD49e | BD Pharmingen (CA) | Mouse IgG1, kappa | 11A1 |
| CD81 | BD Pharmingen (CA) | Mouse IgG1, kappa | JS-81 |
| CD90 | BD Pharmingen (CA) | Mouse IgG1, kappa | 5E10 |
| CD95 | BD Pharmingen (CA) | Mouse IgG1, kappa | DX2 |
| CD124 | BD Pharmingen (CA) | Mouse IgG1, kappa | hIL4R-M57 |
| CD138 | Miltenyi Biotec | Mouse IgG1 | B-B4 |
| CD151 | BD Pharmingen (CA) | Mouse IgG1, kappa | 14A2.H1 |
| CD 166 | BD Pharmingen (CA) | Mouse IgG1, kappa | 3A6 |
| CD 184 | BD Pharmingen (CA) | Mouse IgG2B, kappa | 12G5 |
| CD105 (endoglin) | Santa Cruz Biotechnology (CA) | Mouse IgG1 | P3D1 |
| CD117 (c-Kit) | BD Pharmingen (CA) | Mouse IgG1, kappa | YB5.B8 |
| CD133 | Miltenyi Biotec (CA) | Mouse IgG1 | Ac133 |
| CD44 | BD Pharmingen (CA) | Mouse IgG2b, Kappa | G44-26 |
| CD45 | BD Pharmingen (CA) | Mouse IgG1, Kappa | Hi30 |
| CD49f | BD Pharmingen (CA) | Rat IgG2A, Kappa | G0H3 |
| CD56 (NCAM) | BD Pharmingen (CA) | Mouse IgG1, Kappa | B159 |
| CD73 | BD Pharmingen (CA) | Mouse IgG1, Kappa | AD2 |
| CD90 | BD Pharmingen (CA) | Mouse IgG1, kappa | 5E10 |
| CD95 | BD Pharmingen (CA) | Mouse IgG1, Kappa | DX2 |
| Epithelial adhesion | BD Pharmingen (CA) | Mouse IgG1 | EBA-1 |
| molecule (EpCAM) | | | |
| Hepatocyte growth factor receptor (HGF or c-Met) | R&D systems (MN) | Mouse IgG2A | 95309 |
| Par2 | Santa Cruz Biotechnology (CA) | Mouse IgG2A | Sam11 |
| Platelet/endothelial cell adhesion molecule-1 (PECAM-1) | Santa Cruz Biotechnology (CA) | Mouse IgG1 | WM-59 |
| Thrombin | Santa Cruz Biotechnology (CA) | Mouse IgG1 | ATAP2 |

**TABLE II B. LIST OF SECONDARY CONJUGATED ANTIBODIES USED FOR FACS ANALYSIS**

| Secondary conjugated antibody | Supplier | Dilution |
|---|---|---|
| Goat Anti-Mouse IgG APC conjugated | Jackson ImmunoResearch (PA) | 1:200 |
| Goat Anti-Mouse IgG PE conjugated | Jackson ImmunoResearch (PA) | 1:200 |
| Donkey anti-rabbit PE or - APC conjugated | Jackson ImmunoResearch (PA) | 1:200 |
| Donkey anti-goat PE or - APC conjugated | Jackson ImmunoResearch (PA) | 1:200 |

**TABLE II C. LIST OF ANTIBODIES USED FOR IDENTIFICATION OF INTRACELLULAR MARKERS**

| Antibody | Supplier | Isotype | Clone |
|---|---|---|---|
| Nestin | R&D systems (MN) | Mouse IgG1 | HSG02 |
| Cytokeratin 5/8 | Santa Cruz Biotechnology (CA) | Mouse IgG1 | C50 |
| Vimentin | Santa Cruz Biotechnology (CA) | Mouse IgG1 | V9 |
| Pan-Cytokeratin (4, 5, 6, 8, 10, 13, 18) | Santa Cruz Biotechnology (CA) | Mouse IgG1 | C11 |
| Peripherin | Santa Cruz Biotechnology (CA) | Goat Polyclonal | C19 |
| Gilial fibrillary acidic protein (GFAP) | Santa Cruz Biotechnology (CA) | Goat polyclonal | N-18 |
| Pan-Cytokeratin (14, 15, 16, and 19) | BD Pharmingen (CA) | Mouse IgG1, Kappa | KA4 |
| Beta III tubulin | Chemicon International (CA) | Mouse IgG1 | TU-20 |

**TABLE III. EXPRESSION LEVEL OF SURFACE RECEPTORS OF ADULT PANCREATIC-DERIVED STROMAL CELLS AT PASSAGE 10.**

| Level of expression was assigned based on the magnitude of the geometric mean of the population as compared to isotype controls. "+" refers to a population which has a geometric mean level of at least 10X higher than an isotype matched control and "-" refers to a population which has a geometric mean of level within 3X of the isotype matched control. | |
|---|---|
| Marker | Level of expression |
| ABCG2 | - |
| Alkaline Phosphatase | + |
| CD105 (Endoglin) | + |
| CD117 (C-Kit) | - |
| CD133 | - |
| CD44 | + |
| CD45 | - |
| CD49f | - |
| CD56 (NCAM) | - |
| CD73 (GPI glycoprotein) | + |
| CD90 (Thy-1) | + |
| CD95 (Fas receptor) | + |
| CD151 | + |
| EpCAM | - |
| HGF receptor (c-Met) | - |
| PECAM (CD 31) | - |

**TABLE IV. EXPRESSION LEVEL OF INTERNAL MARKERS OF ADULT PANCREATIC-DERIVED STROMAL CELLS AT PASSAGE 10.**

| Level of expression was assigned based on the magnitude of the geometric mean of the population as compared to isotype controls. "+" refers to a population which has a geometric mean level of at least 10X higher than an isotype matched control and "-" refers to a population which has a geometric mean of level within 3X of the isotype matched control. | |
|---|---|
| Marker | Level of expression |
| Pan cytokeratin: 5, 6, 8, 10, 13, 14, 15, 16, 18, 19 | - |
| GFAP | - |
| Alpha-smooth muscle actin | + |
| MHC | - |
| Peripherin | - |
| Beta III tubulin | + |
| Vimentin | + |
| Nestin | + |

**TABLE V A. LIST OF ANTIBODIES USED FOR IMUNOFLUORESCENCE STAINING.**

| Primary Antibody | Supplier | Isotype | Concentration | Clone |
|---|---|---|---|---|
| Beta III tubulin | R&D Systems (MN) | Mouse IgG2A | 5 µg/ml | Tuj-1 |
| Nestin | R&D Systems (MN) | Mouse IgG1 | 5 µg/ml | 196908 |
| Alpha Smooth muscle actin (SMA) | Sigma (MO) | Mouse IgG2A | 5 µg/ml | 1A4 |
| C-Peptide | Linco (MO) | Rabbit IgG | 1:100 | |
| Amylase | Santa Cruz (CA) | Goat IgG | 1:100 | C-20 |
| GATA4 | Santa Cruz (CA) | Mouse IgG2A | 1:100 | G-4 |
| Prohormone Convertase 1,3 | US Biological (MA) | Rabbit Serum | 1:100 | |
| HNF3β | Santa Cruz (CA) | Goat IgG | 1:100 | M-20 |
| c-Met | | | | |
| E-Cadherin | | | | |
| Gilial fibrillary acidic protein (GFAP) | Chemicon International (CA) | Rabbit serum | 1:500 | |
| Beta III tubulin | Chemicon International (CA) | Mouse IgG1 | 1:100 | TU-20 |
| Neurofilament M-145 kD (NFM) | Chemicon International (CA) | Rabbit serum | 1:100 | |
| Vimentin | Sigma (MO) | Goat IgG | 1:100 | |
| Insulin | Santa Cruz (CA) | Rabbit IgG | 1:100 | H-86 |
| Glucagon | Chemicon International (CA) | Rabbit serum | 1:100 | |
| Somatostatin | Dako (CA) | Rabbit IgG | 1:100 | |
| PDX-1 | Santa Cruz (CA) | Goat IgG | 1:100 | N-18 |
| Glut-2 | Santa Cruz (CA) | Goat IgG | 1:100 | C-19 |
| Glucokinase | Santa Cruz (CA) | Rabbit IgG | 1:100 | H-88 |
| Amylase | Santa Cruz (CA) | Goat IgG | 1:100 | C-20 |
| Cytokeratin 7 | Sigma (MO) | Mouse IgG1 | 1:100 | LDS-68 |
| Cytokeratin 19 | Sigma (MO) | Mouse IgG1 | 1:100 | A53-B/A2 |
| Pan Cytokeratin | Santa Cruz (CA) | Mouse IgG1 | 1:100 | C-11 |
| Microtubule associated protein (MAP2) | Chemicon International (CA) | Rabbit serum | 1:100 | |

**TABLE V B. SECONDARY CONJUGATED ANTIBODIES USED FOR IMMUNOSTAINING.**

| Secondary conjugated antibody | Supplier | Dilution |
|---|---|---|
| Goat Anti-Mouse IgG Alexa Fluor 488 or -594 | Molecular Probes (OR) | 1:200 |
| Donkey anti-goat IgG Alexa Fluor 488 or-594 | Molecular Probes (OR) | 1:200 |
| Chicken anti-Mouse IgG 488, -594 | Molecular Probes (OR) | 1:200 |
| Chicken anti-Goat IgG 488, -594 | Molecular Probes (OR) | 1:200 |
| Chicken anti-Rabbit IgG 488, -594 | Molecular Probes (OR) | 1:200 |
| Rabbit anti-mouse IgG 488, -594 | Molecular Probes (OR) | 1:200 |

**TABLE VI. CELL GROWTH CHARACTERISTICS OF ADULT PANCREATIC-DERIVED STROMAL CELL LINES**

| **Cell Line** | **Media** | **Projected cell # @ Elective Termination** | **Total # of PD** | **Total PDT (hrs)** |
|---|---|---|---|---|
| H4F3 | 2DDHL | 1.87E+24 | 59.3 | 94.3 |
| H4F3 | DMEM | 5.52E+12 | 19.3 | 155.3 |
| H4F3 | CMRL | 2.55E+14 | 24.5 | 122.2 |
| H5F3 | 2DDHL | 9.31E+22 | 55.6 | 63.0 |
| H5F3 | DMEM | 1.81E+26 | 67.3 | 52.4 |
| H6F3 | 2DDHL | 3.53E+17 | 36.5 | 76.9 |
| H7F3 | 2DDHL | 8.66E+12 | 22.0 | 145.4 |
| H8F3 | DMEM | 3.79E+17 | 34.3 | 70.1 |
| H8F3 | CMRL | 1.21E+16 | 28.5 | 85.2 |
| H9F3 | DMEM | 2.37E+16 | 31.1 | 48.7 |
| H9F3 | CMRL | 1.08E+13 | 19.7 | 85.3 |
| | | | | |
| H4F4 | 2DDHL | 1.10E+14 | 25.0 | 154.3 |
| H4F4 | DMEM | 3.51E+12 | 19.3 | 155.5 |
| H4F4 | CMRL | 6.83E+11 | 17.2 | 175.7 |
| H5F4 | 2DDHL | 1.58E+20 | 47.4 | 70.3 |
| H5F4 | DMEM | 2.07E+24 | 60.8 | 64.7 |
| H6F4 | 2DDHL | 1.84E+14 | 26.5 | 152.4 |
| H7F4 | 2DDHL | 2.15E+09 | 10.2 | 231.5 |
| H8F4 | DMEM | 1.68E+18 | 33.3 | 85.1 |
| H9F4 | DMEM | 9.78E+14 | 25.0 | 54.8 |
| | | | | |
| H4F5 | 2DDHL | 1.69E+14 | 26.1 | 136.8 |
| H4F5 | DMEM | 2.50E+17 | 37.1 | 164.3 |
| H4F5 | CMRL | 1.76E+15 | 30.2 | 144.4 |
| H5F5 | DMEM | 7.92E+23 | 58.1 | 68.2 |
| H6F5 | 2DDHL | 2.28E+15 | 31.6 | 93.4 |
| H8F5 | DMEM | 1.14E+17 | 33.6 | 84.2 |
| H9F5 | DMEM | 4.54E+12 | 18.4 | 45.7 |
| | | | | |
| H5F5B1 | DMEM | 1.27E+20 | 44.9 | 72.1 |
| H5F5B2 | DMEM | 3.47E+21 | 49.4 | 67.6 |
| H5F5B3 | DMEM | 2.30E+21 | 49.1 | 65.1 |
| H5F5B4 | DMEM | 3.74E+18 | 39.8 | 75.4 |
| H5F5B5 | DMEM | 2.68E+19 | 43.0 | 67.5 |
| H5F5B6 | DMEM | 9.79E+17 | 38.6 | 72.7 |
| H5F5B7 | DMEM | 1.47E+18 | 39.6 | 67.9 |
| H5F5B8 | DMEM | 4.16E+17 | 37.2 | 71.0 |
| H5F5B9 | DMEM | 4.62E+18 | 41.1 | 64.3 |
| H5F5B10 | DMEM | 1.49E+19 | 42.6 | 55.3 |
| H5F5B11 | DMEM | 1.08E+18 | 39.2 | 58.2 |
| H5F5B12 | DMEM | 1.68E+17 | 36.4 | 60.7 |
| H5F5B13 | DMEM | 1.46E+17 | 36.0 | 46.6 |
| H5F5B14 | DMEM | 1.85E+17 | 36.0 | 52.7 |
| H5F5B15 | DMEM | 2.79E+15 | 30.4 | 56.9 |
| H5F5B16 | DMEM | 2.23E+14 | 26.3 | 57.4 |
| H5F5B17 | DMEM | 6.83E+14 | 28.4 | 51.5 |
| H5F5B18 | DMEM | 7.44E+13 | 24.9 | 49.2 |
| H5F5B19 | DMEM | 3.35E+13 | 24.0 | 46.0 |
| H5F5B20 | DMEM | 4.44E+12 | 20.8 | 38.0 |

**TABLE VII. GENE EXPRESSION FOLLOWING SEQUENTIAL DIFFERENTIATION OF ADULT PANCREATIC-DERIVED STROMAL CELLS**

| | **HNF3B** | | **PDX-1** | | **NKX6.1** | | **INS** | |
|---|---|---|---|---|---|---|---|---|
| **Cell Description** | **Sample Ct** | **% Pancreas** | **Sample Ct** | **% Pancreas** | **Sample Ct** | **% Pancreas** | **Sample Ct** | **% Pancreas** |
| **H5F3P12 - d1 untreated** | **-** | **-** | **-** | **-** | **37.6** | **0.01** | **-** | **-** |
| **H5F3P12 - 1uM MG 132 x 1d** | **-** | **-** | **-** | **-** | **36.6** | **0.02** | **-** | **-** |
| **H5F3P12 - 10uM MG 132 x 1d** | **-** | **-** | **-** | **-** | **33.5** | **0.29** | **-** | **-** |
| **H5F3P12 - 1.25uM TSA x 1d** | **-** | **-** | **-** | **-** | **36.8** | **0.02** | **-** | **-** |
| **H5F3P12 - 2.5uM TSA x 1d** | **34.8** | **0.02** | **34.0** | **0.13** | **30.2** | **2.76** | **-** | **-** |
| **H5F3P12 - d2 untreated** | **-** | **-** | **-** | **-** | **39.1** | **0.01** | **-** | **-** |
| **H5F3P12 - 1uM MG132 x 1d + 1.25uM TSA x 1d** | **34.3** | **0.01** | **34.1** | **0.05** | **29.5** | **1.61** | **-** | **-** |
| **H5F3P12 - 10uM MG132 x 1d + 1.25uM TSA x 1d** | **32.6** | **0.09** | **32.9** | **0.27** | **28.8** | **6.65** | **35.9** | **1.77E-05** |
| **H5F3P12 -1 uM MG132 x 1d + 2.5uM TSA x 1d** | **34.3** | **0.01** | **33.2** | **0.10** | **29.6** | **1.90** | **-** | **-** |
| **H5F3P12 - 10uM MG132 x 1d + 2.5uM TSA x 1d** | **33.6** | **0.03** | **31.9** | **0.35** | **30.9** | **1.05** | **-** | **-** |
| | | | | | | | | |

| | **HNF3B** | | **PDX-1** | | **NKX6.1** | | **INS** | |
|---|---|---|---|---|---|---|---|---|
| **Cell Description** | **Sample Ct** | **% Pancreas** | **Sample Ct** | **% Pancreas** | **Sample Ct** | **% Pancreas** | **Sample Ct** | **% Pancreas** |
| **H5F3P19 - d1 untreated** | **-** | **-** | **-** | **-** | **-** | **-** | **-** | **-** |
| **H5F3P19 - 1uM MG 132 x 1d** | **-** | **-** | **-** | **-** | **-** | **-** | **-** | **-** |
| **H5F3P19 - 10uM MG 132 x 1d** | **-** | **-** | **-** | **-** | **36.3** | **0.04** | **-** | **-** |
| **H5F3P19 - 1.25uM TSA x 1d** | **34.1** | **0.05** | **34.6** | **0.08** | **30.6** | **3.38** | **-** | **-** |
| **H5F3P19 - 2.5uM TSA x 1d** | **32.9** | **0.08** | **32.6** | **0.19** | **29.3** | **5.46** | **-** | **-** |
| **H5F3P19 - d2 untreated** | **-** | **-** | **-** | **-** | **-** | **-** | **-** | **-** |
| **H5F3P19 - 1uM MG132 x 1d + 1.25uM TSA x 1d** | **33.8** | **0.05** | **32.8** | **0.22** | **29.7** | **5.29** | **-** | **-** |
| **H5F3P19 - 10uM MG132 x 1d + 1.25uM TSA x 1d** | **31.7** | **0.69** | **32** | **1.10** | **29.7** | **15.83** | **-** | **-** |
| **H5F3P19 -1 uM MG132 x 1d + 2.5uM TSA x 1d** | **33.4** | **0.07** | **32.3** | **0.30** | **29.1** | **7.98** | **-** | **-** |
| **H5F3P19 - 10uM MG132 x 1d + 2.5uM TSA x 1d** | **31.2** | **0.92** | **31.6** | **1.41** | **29.4** | **18.33** | **-** | **-** |

| | | | | | | | | |
|---|---|---|---|---|---|---|---|---|
| TSA = trichostatin A, 1d = 1 day H = donor pancreas number, F = Fraction number, P = passage number | | | | | | | | |

**TABLE VIII. GENE EXPRESSION FOLLOWING SEQUENTIAL DIFFERENTIATION OF ADULT PANCREATIC-DERIVED STROMAL CELLS**

| | **HNF3B** | | **PDX-1** | | **NKX6.1** | |
|---|---|---|---|---|---|---|
| **Cell Description** | **Sample Ct** | **% of Pancreas** | **Sample Ct** | **% of Pancreas** | **Sample Ct** | **% of Pancreas** |
| **H5F3P18 - d1 untreated** | **-** | **-** | **-** | **-** | **-** | **-** |
| **H5F3P18 - 10 uM B5306 x 1d** | **-** | **-** | **-** | **-** | **-** | **-** |
| **H5F3P18 - d2 untreated** | **-** | **-** | **-** | **-** | **-** | **-** |
| **H5F3P18 - DMEM x 1d + 1.25 TSA x 1d** | **32.8** | **0.59** | **33.8** | **1.18** | **30.9** | **18.17** |
| **H5F3P18 - 10 uM B5306 x 1d + 1.25 uM TSA x 1d** | **33.1** | **0.14** | **34.5** | **0.22** | **30.9** | **5.56** |
| **H5F3P18 - 10 uM B5306 x 1d + 2.5 uM TSA x 1d** | **33.3** | **0.17** | **34.0** | **0.41** | **30.8** | **8.42** |
| **H5F3P18 - d3 untreated** | **-** | **-** | **-** | **-** | **-** | **-** |
| **H5F3P18 - 10 uM B5306 x 3d** | **-** | **-** | **-** | **-** | **-** | **-** |
| **H5F3P18 - d7 untreated** | **-** | **-** | **-** | **-** | **-** | **-** |
| **H5F3P18 - 10 uM B5306 x 7d** | **-** | **-** | **-** | **-** | **-** | **-** |
| **H5F3P18 - d8 untreated** | **-** | **-** | **-** | **-** | **-** | **-** |
| **H5F3P18 - DMEM x 7d + 1.25 uM TSA x 1d** | **31** | **0.09** | **31.5** | **0.24** | **28.2** | **3.46** |
| **H5F3P18 - DMEM x 7d + 2.5 uM TSA x 1d** | **31.1** | **0.12** | **31.4** | **0.37** | **28.4** | **4.51** |
| **H5F3P18 - 10 uM B5306 x 7d + 1.25 uM TSA x 1d** | **31.9** | **0.07** | **32.5** | **0.17** | **28.4** | **4.16** |
| **H5F3P18 - 10 uM B5306 x 7d + 2.5 uM TSA x 1d** | **31.1** | **0.06** | **31.3** | **0.20** | **28** | **2.81** |
| **H5F3P18 - d10 untreated** | **-** | **-** | **-** | **-** | **-** | **-** |
| **H5F3P18 - 10 uM B5306 x 10d** | **-** | **-** | - | **-** | **-** | **-** |
| **H5F3P18 - d14 untreated** | **-** | **-** | **-** | **-** | **-** | **-** |
| **H5F3P18 - 10 uM B5306 x 14d** | **-** | **-** | - | **-** | **-** | **-** |
| **H5F3P18 - d15 untreated** | **-** | **-** | **-** | **-** | **-** | **-** |
| **H5F3P18 - DMEM x 14d + 1.25 uM TSA x 1d** | **33.8** | **0.07** | **33.7** | **0.14** | **30.1** | **2.39** |
| **H5F3P18 - DMEM x 14d + 2.5 uM TSA x 1d** | **-** | **-** | **-** | **-** | **na** | **na** |
| **H5F3P18 - 10 uM B5306 x 14d + 1.25 uM TSA x 1d** | **35.3** | **0.04** | **34.9** | **0.08** | **30.5** | **2.60** |
| **H5F3P18 - 10 uM B5306 x 14d + 2.5 uM TSA x 1d** | **34.9** | **0.03** | **33.7** | **0.14** | **29.9** | **2.78** |

| | | | | | | |
|---|---|---|---|---|---|---|
| TSA = trichostatin A, 1d = 1 day H = donor pancreas number, F = Fraction number, P = passage number | | | | | | |

**TABLE IX. MEDIA FORMULATIONS FOR ADULT PANCREATIC-DERIVED STROMAL CELL DIFFERENTIATION**

| | | | |
|---|---|---|---|
| DM #1 | DMEM HG | DM #6 | DMEM HG |
| | 1 % FBS | | 1 % FBS |
| | Activin A - 20 ng/mL | | EGF - 0.3 ug/mL |
| | Betacellulin - 10 ng/mL | | Gastrin - 1.0 ug/mL |
| | bFGF - 20 ng/mL | | |
| | GLP-1 R agonist - 50 nM | DM #7 | DMEM HG |
| | Nicotinamide - 10 mM | | 1 % FBS |
| | | | EGF 0.3 ug/mL |
| DM #2 | DMEM - LG | | Gastrin 1.0 ug/mL |
| | 1 % FBS | | GLP-1 R agonist - 50 nM |
| | Activin A - 20 ng/mL | | Nicotinamide - 10 mM |
| | Betacellulin - 10 ng/mL | | |
| | bFGF - 20 ng/mL | DM #8 | DMEM/F12 |
| | GLP-1 R agonist - 50 nM | | B27 |
| | Nicotinamide - 10 mM | | N2 |
| | | | EGF - 0.3 ug/mL |
| DM #3 | DMEM/F12 | | Gastrin 1.0 ug/mL |
| | N2 | | Exendin 4 - 20 nM |
| | B27 | | Nicotinamide - 10 mM |
| | Laminin 1 ug/mL | | |
| | bFGF - 20 ng/mL | DM #9 | DMEM/F12 |
| | Nicotinamide 10 mM | | B27 |
| | | | Exendin 4 - 20 nM |
| DM #4 | DMEM/F12 | | Nicotinamide - 10 mM |
| | B27 | | |
| | bFGF - 20 ng/mL | DM #10 | CMRL - 1066 |
| | EGF - 20 ng/mL | | 1 % BSA |
| | | | ITS-x |
| DM #5 | DMEM/F12 | | Na Pyruvate |
| | B27 | | |
| | GLP-1 R agonist - 50 nM | | |
| | Nicotinamide - 10 mM | | |

**TABLE X. PCR RESULTS FROM DIFFERENTIATION OF FRACTION3 PASSAGE 5 ADULT PANCREATIC-DERIVED STROMAL CELLS WITH DIFFERENTIATION MEDIA DM#1 - DM#10.**

| **Differentiation Media** | **2 week differentiation** | **Aggregation** |
|---|---|---|
| | **Gluc = 37** | |
| **DM #1** | **Nkx6.1 = 36** | |
| | **Pax6 = 34** | **INS = 36** |
| | **Somat = 34** | **HNF3B = 27** |
| | **Nkx6.1 = 36** | |
| **DM #2** | **Pax6 = 35** | |
| | **Somat =36** | **PDX = 36** |
| | **Ins = 31** | |
| **DM #3** | **Nkx6.1 = 37** | |
| | **Pax6 = 34** | |
| | **Somat = 38** | **ND** |
| | **Ins = 33** | |
| **DM #4** | **Pax6 = 36** | |
| | **Somat = 37** | **ND** |
| **DM #5** | **Pax6 = 34** | **ND** |
| **DM #6** | | **PDX = 35** |
| | **Pax6 = 35** | **HNF3B = 38** |
| | | **PDX = 34** |
| **DM #7** | | **INS = 35** |
| | **Pax6 = 34** | **HNF3B = 30** |
| **DM #8** | **Pax6 = 34** | **ND** |
| **DM #9** | **Pax6 = 33** | **ND** |
| **DM #10** | **Nkx6.1 = 37** | **PDX = 35** |
| | **Pax6 = 33** | **HNF3B = 28** |

| | | |
|---|---|---|
| **ND = Not Done** | | |

**TABLE XI. PCR RESULTS FROM DIFFERENTIATION OF FRACTION 3 PASSAGE 13 ADULT PANCREATIC-DERIVED STROMAL CELLS WITH DIFFERENTIATION MEDIA DM#1 - DM#10.**

| **Differentiation Media** | **2 week Differentiation** | **Aggregation** |
|---|---|---|
| **DM #1** | | **Nkx6.1 = 38** |
| | **all negative** | **Somat = 33** |
| **DM #2** | **all negative** | **Somat = 33** |
| **DM #3** | **all negative** | **all negative** |
| **DM #4** | **all negative** | **ND** |
| **DM #5** | **all negative** | **ND** |
| **DM #6** | **all negative** | **all negative** |
| **DM #7** | | **Ins = 36** |
| | | **Pax6 = 36** |
| | **all negative** | **Somat = 34** |
| **DM #8** | **all negative** | **ND** |
| **DM #9** | **all negative** | **ND** |
| **DM #10** | **all negative** | **ND** |

| | | |
|---|---|---|
| **ND = Not Done** | | |

**TABLE XII: DISTRIBUTION OF CHROMASOME NUMBERS IN THE 100 METAPHASES ANALYZED IN A REPRESENTATIVE ADULT PANCREATIC-DERIVED STROMAL CELL LINE.**

| Number of chromosomes | Number of metaphases |
|---|---|
| 45 | 4 |
| 46 | 96 |

**TABLE XIII. CHROMASOME ABBERATION DATA FOR A REPRESENTATIVE ADULT PANCREATIC-DERIVED STROMAL CELL LINE.**

| Type of Aberration | | | | | | |
|---|---|---|---|---|---|---|
| Chromatid | | Chromosome | | Severely damaged cell | Number of cells aberrant | Total number of aberrations |
| Deletion | Insertion | Deletion | Insertion | | | |
| 0 | 0 | 0 | 0 | 0 | 0 | 0 |

**TABLE XIV: GENES THAT WERE DIFFERENTIALLY EXPRESSED AT LEAST 5-FOLD BETWEEN ADULT PANCREATIC-DERIVED STROMAL CELLS DERIVED FROM THE F3 AND F5 FRACTIONS OF A DONOR HUMAN PANCREAS.**

| Gene Identifier | Gene Name | Ratio (F3/F5) Direction | adj. p-value |
|---|---|---|---|
| NM_033439 | Homo sapiens chromosome 9 open reading frame 26 (NF-HEV) (C9orf26), mRNA | 169.79 Up | 2.17E-04 |
| NM_015973 | Homo sapiens galanin (GAL), mRNA | 67.54 Up | 2.09E-05 |
| NM_005525 | Homo sapiens hydroxysteroid (11-beta) dehydrogenase 1 (HSD11B1), transcript variant 1, mRNA | 37.62 Up | 1.30E-05 |
| NM_007193 | Homo sapiens annexin A10 (ANXA10), mRNA | 27.82 Up | 1.02E-04 |
| NM_005525 | Homo sapiens hydroxysteroid (11-beta) dehydrogenase 1 (HSD11B1), transcript variant 1, mRNA | 24.43 Up | 1.25E-04 |
| NM_014178 | Homo sapiens syntaxin binding protein 6 (amisyn) (STXBP6), mRNA | 20 Up | 1.81E-05 |
| NM_002422 | Homo sapiens matrix metalloproteinase 3 (stromelysin 1, progelatinase) (MMP3), mRNA | 16.5 Up | 4.37E-05 |
| NM_000640 | Homo sapiens interleukin 13 receptor, alpha 2 (IL13RA2), mRNA | 15.93 Up | 2.28E-04 |
| NM_018894 | Homo sapiens EGF-containing fibulin-like extracellular matrix protein 1 (EFEMP1), transcript variant 2, mRNA | 14.85 Up | 1.25E-04 |
| NM_000735 | Homo sapiens glycoprotein hormones, alpha polypeptide (CGA), mRNA | 14.7 Up | 1.81E-05 |
| NM_000584 | Homo sapiens interleukin 8 (IL8), mRNA | 13.7 Up | 1.97E-03 |
| NM_005807 | Homo sapiens proteoglycan 4 (PRG4), mRNA | 12.53 Up | 6.38E-04 |
| BE904671 | 601498784F1 NIH_MGC_70 Homo sapiens cDNA clone IMAGE:3900717 5, mRNA sequence | 12.38 Up | 3.79E-04 |
| NM_001086 | Homo sapiens arylacetamide deacetylase (esterase) (AADAC), mRNA | 11.98 Up | 1.01E-03 |
| NM_018371 | Homo sapiens chondroitin beta1,4 N-acetylgalactosaminyltransferase (ChGn), mRNA | 11.9 Up | 6.00E-04 |
| NM_053044 | Homo sapiens serine protease HTRA3 (HTRA3), mRNA | 11.09 Up | 3.46E-04 |
| D29453 | HUMNK566 Human epidermal keratinocyte Homo sapiens cDNA clone 566, mRNA sequence | 10.25 Up | 1.24E-04 |
| R41565 | yf88e01.s1 Soares infant brain 1NIB Homo sapiens cDNA clone IMAGE:29531 3, mRNA sequence | 10.23 Up | 8.47E-05 |
| NM_005127 | Homo sapiens C-type (calcium dependent, carbohydrate-recognition domain) lectin, superfamily member 2 (activation-induced) (CLECSF2), mRNA | 10.11 Up | 8.47E-05 |
| NM_002089 | Homo sapiens chemokine (C-X-C motif) ligand 2 (CXCL2), mRNA | 9.87 Up | 6.92E-04 |
| NM_012310 | Homo sapiens kinesin family member 4A (KIF4A), mRNA | 9.85 Up | 1.10E-03 |
| AI215024 | qg66e11.x1 Soares_testis_NHT Homo sapiens cDNA clone IMAGE:1840172 3, mRNA sequence | 9.43 Up | 1.48E-04 |
| NM_153256 | Homo sapiens chromosome 10 open reading frame 47 (C10orf47), mRNA | 9.34 Up | 2.44E-04 |
| NM_005951 | Homo sapiens metallothionein 1H (MT1H), mRNA | 9.09 Up | 1.79E-04 |
| NM_006211 | Homo sapiens proenkephalin (PENK), mRNA | 8.78 Up | 1.30E-03 |
| NM_198538 | Homo sapiens HLAR698 (UNQ698), mRNA | 8.72 Up | 3.45E-04 |
| NM_006183 | Homo sapiens neurotensin (NTS), mRNA | 8.19 Up | 3.72E-04 |
| BU536871 | AGENCOURT_10224340 NIH_MGC_141 Homo sapiens cDNA clone IMAGE:6565454 5, mRNA sequence | 8.16 Up | 1.23E-03 |
| NM_000693 | Homo sapiens aldehyde dehydrogenase 1 family, member A3 (ALDH1A3), mRNA | 7.99 Up | 1.56E-03 |
| NM_005950 | Homo sapiens metallothionein 1G (MT1G), mRNA | 7.89 Up | 2.51E-03 |
| NM_145244 | Homo sapiens DNA-damage-inducible transcript 4-like (DDIT4L), mRNA | 7.8 Up | 2.17E-04 |
| BF514016 | UI-H-BW1-amv-f-04-0-UI.s1 NCI_CGAP_Sub7 Homo sapiens cDNA clone IMAGE:3071359 3, mRNA sequence | 7.73 Up | 5.09E-05 |
| NM_004675 | Homo sapiens ras homolog gene family, member I (ARHI), mRNA | 7.71 Up | 2.32E-03 |
| NM_017805 | Homo sapiens Ras interacting protein 1 (RASIP1), mRNA | 7.66 Up | 4.22E-04 |
| AA662240 | nu89c01.s1 NCI_CGAP _Alv1 Homo sapiens cDNA clone IMAGE:1217856, mRNA sequence | 7.44 Up | 2.30E-04 |
| NM_002426 | Homo sapiens matrix metalloproteinase 12 (macrophage elastase) (MMP12), mRNA | 7.41 Up | 2.28E-03 |
| NM_000170 | Homo sapiens glycine dehydrogenase (decarboxylating; glycine decarboxylase, glycine cleavage system protein P) (GLDC), mRNA | 7.35 Up | 1.53E-04 |
| CB160856 | K-EST0220612 L18POOL1n1 Homo sapiens cDNA clone L18POOL1n1-33-F12 5, mRNA sequence | 7.33 Up | 2.17E-04 |
| NM_032849 | Homo sapiens hypothetical protein FLJ14834 (FLJ14834), mRNA | 7.07 Up | 1.75E-04 |
| BM988338 | UI-H-DH0-asd-f-10-0-UI.s1 NCI_CGAP_DH0 Homo sapiens cDNA clone IMAGE:5857545 3, mRNA sequence | 7.01 Up | 4.12E-04 |
| NM_145033 | Homo sapiens chromosome 21 open reading frame 100 (C21orf100), mRNA | 6.91 Up | 8.47E-05 |
| NM_033120 | Homo sapiens naked cuticle homolog 2 (Drosophila) (NKD2), mRNA | 6.83 Up | 2.17E-04 |
| NM_080388 | Homo sapiens S100 calcium binding protein A16 (S100A16), mRNA | 6.67 Up | 2.13E-03 |
| AA813769 | ai69h12.s1 Soares_testis_NHT Homo sapiens cDNA | 6.62 Up | 1.48E-04 |
| | clone 1376135 3, mRNA sequence | | |
| NM_198389 | Homo sapiens lung type-I cell membrane-associated glycoprotein (T1A-2), transcript variant 2, mRNA | 6.55 Up | 2.78E-04 |
| AK024865 | Homo sapiens cDNA: FLJ21212 fis, clone COL00502 | 6.54 Up | 2.34E-04 |
| AW294090 | UI-H-BI2-ahg-b-12-0-UI.s1 NCI_CGAP _Sub4 Homo sapiens cDNA clone IMAGE:2726734 3, mRNA sequence | 6.53 Up | 2.19E-04 |
| AA553336 | nk61e11.s1 NCI_CGAP_Sch1 Homo sapiens cDNA clone IMAGE:1018028 3, mRNA sequence | 6.46 Up | 9.15E-04 |
| NM_006273 | Homo sapiens chemokine (C-C motif) ligand 7 (CCL7), mRNA | 6.45 Up | 3.45E-04 |
| BG206063 | RST25498 Athersys RAGE Library Homo sapiens cDNA, mRNA sequence | 6.41 Up | 2.09E-05 |
| NM_022154 | Homo sapiens solute carrier family 39 (zinc transporter), member 8 (SLC39A8), mRNA | 6.28 Up | 1.02E-03 |
| NM_002245 | Homo sapiens potassium channel, subfamily K, member 1 (KCNK1), mRNA | 6.24 Up | 3.68E-04 |
| NM_022833 | Homo sapiens chromosome 9 open reading frame 88 (C9orf88), mRNA | 6.24 Up | 3.27E-04 |
| NM_017779 | Homo sapiens DEP domain containing 1 (DEPDC1), mRNA | 6.04 Up | 2.26E-03 |
| NM_012320 | Homo sapiens lysophospholipase 3 (lysosomal phospholipase A2) (LYPLA3), mRNA | 5.98 Up | 3.46E-04 |
| BM977193 | UI-CF-DU1-ads-h-16-0-UI.s1 UI-CF-DU1 Homo sapiens cDNA clone UI-CF-DU1-ads-h-16-0-UI 3, mRNA sequence | 5.97 Up | 3.45E-04 |
| NM_001928 | Homo sapiens D component of complement (adipsin) (DF), mRNA | 5.89 Up | 9.04E-04 |
| NM_014736 | Homo sapiens KIAA0101 (KIAA0101), mRNA | 5.84 Up | 1.25E-04 |
| NM_002497 | Homo sapiens NIMA (never in mitosis gene a)-related kinase 2 (NEK2), mRNA | 5.81 Up | 2.17E-04 |
| NM_022809 | Homo sapiens cell division cycle 25C (CDC25C), transcript variant 2, mRNA | 5.81 Up | 4.37E-05 |
| NM_138484 | Homo sapiens shugoshin-like 1 (S. pombe) (SGOL1), mRNA | 5.8 Up | 1.24E-04 |
| NM_003975 | Homo sapiens SH2 domain protein 2A (SH2D2A), mRNA | 5.68 Up | 2.46E-04 |
| NM_020675 | Homo sapiens kinetochore protein Spc25 (Spc25), mRNA | 5.68 Up | 1.26E-04 |
| NM_001657 | Homo sapiens amphiregulin (schwannoma-derived growth factor) (AREG), mRNA | 5.62 Up | 2.30E-04 |
| NM_145697 | Homo sapiens cell division cycle associated 1 (CDCA1), transcript variant 1, mRNA | 5.59 Up | 3.75E-04 |
| NM_005213 | Homo sapiens cystatin A (stefin A) (CSTA), mRNA | 5.51 Up | 1.02E-04 |
| AW968578 | EST380654 MAGE resequences, MAGJ Homo sapiens cDNA, mRNA sequence | 5.43 Up | 1.68E-04 |
| NM_002922 | Homo sapiens regulator of G-protein signalling 1 (RGS1), mRNA | 5.38 Up | 7.97E-04 |
| BC044933 | Homo sapiens, clone IMAGE:4540326, mRNA, partial cds | 5.37 Up | 4.22E-04 |
| AA043255 | zk49f07.s1 Soares_pregnant_uterus_NbHPU Homo sapiens cDNA clone IMAGE:486181 3, mRNA sequence | 5.31 Up | 8.47E-05 |
| NM_199414 | Homo sapiens protein regulator of cytokinesis 1 (PRC1), transcript variant 3, mRNA | 5.29 Up | 2.09E-05 |
| AK093618 | Homo sapiens cDNA FLJ36299 fis, clone THYMU2004356 | 5.26 Up | 5.30E-04 |
| NM_152759 | Homo sapiens hypothetical protein MGC35140 (MGC35140), mRNA | 5.23 Up | 1.75E-04 |
| NM_006438 | Homo sapiens collectin sub-family member 10 (C-type lectin) (COLEC10), mRNA | 5.22 Up | 3.45E-04 |
| CA310410 | UI-H-FE1-bei-b-08-0-UI.s2 NCI_CGAP_FE1 Homo sapiens cDNA clone UI-H-FE1-bei-b-08-0-UI 3, mRNA sequence | 5.2 Up | 8.47E-05 |
| NM_020242 | Homo sapiens kinesin-like 7 (KNSL7), mRNA | 5.18 Up | 1.68E-04 |
| AB058769 | Homo sapiens mRNA for KIAA1866 protein, partial cds | 5.18 Up | 4.64E-04 |
| AI807813 | wf50h08.x1 Soares_NFL_T_GBC_S1 Homo sapiens cDNA clone IMAGE:2359071 3, mRNA sequence | 5.17 Up | 2.72E-04 |
| NM_018944 | Homo sapiens chromosome 21 open reading frame 45 (C21orf45), mRNA | 5.17 Up | 3.46E-04 |
| NM_018136 | Homo sapiens asp (abnormal spindle)-like, microcephaly associated (Drosophila) (ASPM), mRNA | 5.16 Up | 3.48E-04 |
| NM_001353 | Homo sapiens aldo-keto reductase family 1, member C1 (dihydrodiol dehydrogenase 1; 20-alpha (3-alpha)-hydroxysteroid dehydrogenase) (AKR1C1), mRNA | 5.1 Up | 8.62E-05 |
| NM_001809 | Homo sapiens centromere protein A, 17kDa (CENPA), mRNA | 5.02 Up | 4.44E-04 |
| NM_002009 | Homo sapiens fibroblast growth factor 7 (keratinocyte growth factor) (FGF7), mRNA | 5 Up | 2.32E-03 |
| NM_002474 | Homo sapiens myosin, heavy polypeptide 11, smooth muscle (MYH11), transcript variant SM1, mRNA | 18.05 Down | 1.02E-04 |
| BG571477 | 602592765F1 NIH_MGC_79 Homo sapiens cDNA clone IMAGE:4720025 5, mRNA sequence | 13.35 Down | 4.10E-03 |
| NM_206927 | Homo sapiens synaptotagmin-like 2 (SYTL2), transcript variant c, mRNA | 9.94 Down | 3.13E-03 |
| NM_014476 | Homo sapiens PDZ and LIM domain 3 (PDLIM3), mRNA | 9.51 Down | 2.32E-03 |
| AK124751 | Homo sapiens cDNA FLJ42761 fis, clone BRAWH3002574, highly similar to Calpain 2, large [catalytic] subunit precursor (EC 3.4.22.17) | 9.09 Down | 5.54E-04 |
| AF269162 | Homo sapiens c21orf7 form B mRNA, complete cds | 7.75 Down | 1.03E-02 |
| NM_003617 | Homo sapiens regulator of G-protein signalling 5 (RGS5), mRNA | 7.59 Down | 6.56E-03 |
| NM_139211 | Homo sapiens homeodomain-only protein (HOP), transcript variant 2, mRNA | 7.55 Down | 2.34E-04 |
| NM_015234 | Homo sapiens G protein-coupled receptor 116 | 7.47 Down | 1.97E-03 |
| | (GPR116), mRNA | | |
| NM_012278 | Homo sapiens integrin beta 1 binding protein (melusin) 2 (ITGB1BP2), mRNA | 7.05 Down | 1.09E-03 |
| AL713608 | DKFZp686O179_r1 686 (synonym: hlcc3) Homo sapiens cDNA clone DKFZp686O179 5, mRNA sequence | 7.03 Down | 6.29E-03 |
| BQ439091 | AGENCOURT_7761579 NIH_MGC_70 Homo sapiens cDNA clone IMAGE:6020085 5, mRNA sequence | 6.92 Down | 3.27E-04 |
| BX640908 | Homo sapiens mRNA; cDNA DKFZp686J18113 (from clone DKFZp686J18113) | 6.87 Down | 1.03E-03 |
| NM_024586 | Homo sapiens oxysterol binding protein-like 9 (OSBPL9), transcript variant 6, mRNA | 6.69 Down | 1.30E-02 |
| NM_012137 | Homo sapiens dimethylarginine dimethylaminohydrolase 1 (DDAH1), mRNA | 6.22 Down | 1.36E-02 |
| BX119527 | BX119527 Soares_fetal_liver_spleen_1NFLS_S1 Homo sapiens cDNA clone IMAGp998O214529 ; IMAGE:1851116, mRNA sequence | 5.99 Down | 1.94E-03 |
| NM_021154 | Homo sapiens phosphoserine aminotransferase 1 (PSAT1), transcript variant 2, mRNA | 5.83 Down | 2.32E-02 |
| NM_003617 | Homo sapiens regulator of G-protein signalling 5 (RGS5), mRNA | 5.68 Down | 4.22E-04 |
| H08012 | yI91b08.r1 Soares infant brain 1NIB Homo sapiens cDNA clone IMAGE:45474 5, mRNA sequence | 5.54 Down | 1.03E-03 |
| AA608841 | af83g04.s1 Soares_testis_NHT Homo sapiens cDNA clone IMAGE:1048662 3, mRNA sequence | 5.39 Down | 8.01E-03 |
| NM_016203 | Homo sapiens protein kinase, AMP-activated, gamma 2 non-catalytic subunit (PRKAG2), mRNA | 5.38 Down | 5.05E-03 |
| BE697175 | RC1-CT0414-260700-011-a03 CT0414 Homo sapiens cDNA, mRNA sequence | 5.37 Down | 1.89E-03 |
| NM_014799 | Homo sapiens hephaestin (HEPH), transcript variant 2, mRNA | 5.36 Down | 1.11E-02 |
| NM_021229 | Homo sapiens netrin 4 (NTN4), mRNA | 5.35 Down | 4.06E-02 |
| AA844712 | ai70e12.s1 Soares_testis_NHT Homo sapiens cDNA clone IMAGE:1376206 3, mRNA sequence | 5.35 Down | 3.21E-03 |
| NM_006587 | Homo sapiens corin, serine protease (CORIN), mRNA | 5.11 Down | 7.77E-03 |
| NM_021098 | Homo sapiens calcium channel, voltage-dependent, alpha 1H subunit (CACNA1H), transcript variant 1, mRNA | 5.06 Down | 2.80E-02 |

**TABLE XV : GENES THAT WERE DIFFERENTIALLY EXPRESSED AT LEAST 5-FOLD BETWEEN ADULT PANCREATIC-DERIVED STROMAL CELLS DERIVED FROM THE F4 AND F5 FRACTIONS A SINGLE DONOR HUMAN PANCREAS.**

| Gene Identifier | Gene Name | Ratio (F5/F4) Direction | adj. p-value |
|---|---|---|---|
| NM_033439 | Homo sapiens chromosome 9 open reading frame 26 (NF-HEV) (C9orf26), mRNA | 50.81 Up | 2.11E-06 |
| NM_015973 | Homo sapiens galanin (GAL), mRNA | 19.55 Up | 4.25E-05 |
| NM_007193 | Homo sapiens annexin A10 (ANXA10), mRNA | 19.55 Up | 2.77E-05 |
| NM_005525 | Homo sapiens hydroxysteroid (11-beta) dehydrogenase 1 (HSD11B1), transcript variant 1, mRNA | 15.43 Up | 1.78E-05 |
| NM_005525 | Homo sapiens hydroxysteroid (11-beta) dehydrogenase 1 (HSD11B1), transcript variant 1, mRNA | 12.49 Up | 4.38E-05 |
| NM_000170 | Homo sapiens glycine dehydrogenase (decarboxylating; glycine decarboxylase, glycine cleavage system protein P) (GLDC), mRNA | 10.23 Up | 2.77E-05 |
| NM_018371 | Homo sapiens chondroitin beta1,4 N-acetylgalactosaminyltransferase (ChGn), mRNA | 9.72 Up | 1.57E-04 |
| NM_002422 | Homo sapiens matrix metalloproteinase 3 (stromelysin 1, progelatinase) (MMP3), mRNA | 9.43 Up | 1.78E-05 |
| NM_145033 | Homo sapiens chromosome 21 open reading frame 100 (C21orf100), mRNA | 8.73 Up | 4.25E-05 |
| BF514016 | UI-H-BW1-amv-f-04-0-UI.s1 NCI_CGAP_Sub7 Homo sapiens cDNA clone IMAGE:3071359 3, mRNA sequence | 8.16 Up | 5.53E-05 |
| NM_005382 | Homo sapiens neurofilament 3 (150kDa medium) (NEF3), mRNA | 8.15 Up | 1.83E-04 |
| NM_000735 | Homo sapiens glycoprotein hormones, alpha polypeptide (CGA), mRNA | 7.8 Up | 5.48E-04 |
| NM_021992 | Homo sapiens thymosin, beta, identified in neuroblastoma cells (TMSNB), mRNA | 7.79 Up | 6.74E-04 |
| NM_001523 | Homo sapiens hyaluronan synthase 1 (HAS1), mRNA | 7.34 Up | 2.68E-06 |
| NM_145697 | Homo sapiens cell division cycle associated 1 (CDCA1), transcript variant | 7.22 Up | 2.74E-04 |
| | 1, mRNA | | |
| BQ267806 | ij94e04.x1 Human insulinoma Homo sapiens cDNA clone IMAGE:5779278 3, mRNA sequence | 7.2 Up | 2.57E-04 |
| NM_001255 | Homo sapiens CDC20 cell division cycle 20 homolog (S. cerevisiae) (CDC20), mRNA | 7.2 Up | 4.28E-04 |
| NM_017413 | Homo sapiens apelin, AGTRL1 ligand (APLN), mRNA | 7.06 Up | 5.94E-05 |
| NM_005252 | Homo sapiens v-fos FBJ murine osteosarcoma viral oncogene homolog (FOS), mRNA | 6.96 Up | 1.12E-02 |
| CB160856 | K-EST0220612 L18POOL1n1 Homo sapiens cDNA clone L18POOL1n1-33-F12 5, mRNA sequence | 6.83 Up | 6.45E-05 |
| NM_002497 | Homo sapiens NIMA (never in mitosis gene a)-related kinase 2 (NEK2), mRNA | 6.75 Up | 4.25E-05 |
| NM_012310 | Homo sapiens kinesin family member 4A (KIF4A), mRNA | 6.75 Up | 3.90E-03 |
| NM_020675 | Homo sapiens kinetochore protein Spc25 (Spc25), mRNA | 6.72 Up | 4.25E-05 |
| NM_005192 | Homo sapiens cyclin-dependent kinase inhibitor 3 (CDK2-associated dual specificity phosphatase) (CDKN3), mRNA | 6.56 Up | 1.11E-04 |
| NM_006211 | Homo sapiens proenkephalin (PENK), mRNA | 6.56 Up | 3.63E-04 |
| NM_022809 | Homo sapiens cell division cycle 25C (CDC25C), transcript variant 2, mRNA | 6.43 Up | 1.78E-05 |
| NM_152694 | Homo sapiens zinc finger, CCHC domain containing 5 (ZCCHC5), mRNA | 6.43 Up | 2.78E-04 |
| NM_001657 | Homo sapiens amphiregulin (schwannoma-derived growth factor) (AREG), mRNA | 6.41 Up | 9.94E-05 |
| NM_138484 | Homo sapiens shugoshin-like 1 (S. pombe) (SGOL1), mRNA | 6.41 Up | 1.72E-04 |
| NM_001809 | Homo sapiens centromere protein A, 17kDa (CENPA), mRNA | 6.36 Up | 6.90E-05 |
| NM_002421 | Homo sapiens matrix metalloproteinase 1 (interstitial collagenase) (MMP1), mRNA | 6.36 Up | 3.50E-03 |
| NM_024053 | Homo sapiens chromosome 22 open reading frame 18 (C22orf18), transcript variant 1, mRNA | 6.3 Up | 9.40E-04 |
| NM_181803 | Homo sapiens ubiquitin-conjugating enzyme E2C (UBE2C), transcript variant 6, mRNA | 6.11 Up | 4.76E-04 |
| NM_018304 | Homo sapiens hypothetical protein FLJ11029 (FLJ11029), mRNA | 6.09 Up | 9.77E-04 |
| NM_006681 | Homo sapiens neuromedin U (NMU), | 6.07 Up | 1.57E-04 |
| | mRNA | | |
| NM_012484 | Homo sapiens hyaluronan-mediated motility receptor (RHAMM) (HMMR), transcript variant 1, mRNA | 6.03 Up | 2.77E-05 |
| BQ053282 | AGENCOURT_6821603 NIH_MGC_106 Homo sapiens cDNA clone IMAGE:5934939 5, mRNA sequence | 6 Up | 6.90E-05 |
| NM_014736 | Homo sapiens KIAA0101 (KIAA0101), mRNA | 5.98 Up | 2.72E-04 |
| NM_006607 | Homo sapiens pituitary tumor-transforming 2 (PTTG2), mRNA | 5.95 Up | 3.46E-05 |
| AI215024 | qg66e11.x1 Soares_testis_NHT Homo sapiens cDNA clone IMAGE:1840172 3, mRNA sequence | 5.95 Up | 1.51E-04 |
| NM_032117 | Homo sapiens GAJ protein (GAJ), mRNA | 5.89 Up | 4.58E-05 |
| NM_002658 | Homo sapiens plasminogen activator, urokinase (PLAU), mRNA | 5.86 Up | 4.25E-05 |
| BC069212 | Homo sapiens cDNA clone IMAGE:4871934, partial cds | 5.83 Up | 2.77E-05 |
| NM_017779 | Homo sapiens DEP domain containing 1 (DEPDC1), mRNA | 5.79 Up | 4.26E-03 |
| NM_007280 | Homo sapiens Opa-interacting protein 5 (OIP5), mRNA | 5.78 Up | 2.77E-05 |
| NM_001786 | Homo sapiens cell division cycle 2, G1 to S and G2 to M (CDC2), transcript variant 1, mRNA | 5.74 Up | 5.06E-05 |
| NM_018492 | Homo sapiens T-LAK cell-originated protein kinase (TOPK), mRNA | 5.7 Up | 6.90E-05 |
| NM_018454 | Homo sapiens nucleolar and spindle associated protein 1 (NUSAP1), mRNA | 5.7 Up | 1.24E-03 |
| NM_004701 | Homo sapiens cyclin B2 (CCNB2), mRNA | 5.68 Up | 2.41E-04 |
| NM_003155 | Homo sapiens stanniocalcin 1 (STC1), mRNA | 5.66 Up | 6.59E-04 |
| NM_014750 | Homo sapiens discs, large homolog 7 (Drosophila) (DLG7), mRNA | 5.62 Up | 4.17E-04 |
| NM_006461 | Homo sapiens sperm associated antigen 5 (SPAG5), mRNA | 5.58 Up | 5.17E-05 |
| NM_018136 | Homo sapiens asp (abnormal spindle)-like, microcephaly associated (Drosophila) (ASPM), mRNA | 5.54 Up | 3.76E-04 |
| NM_004217 | Homo sapiens aurora kinase B (AURKB), mRNA | 5.48 Up | 2.37E-04 |
| NM_016195 | Homo sapiens M-phase phosphoprotein 1 (MPHOSPH1), mRNA | 5.46 Up | 1.54E-03 |
| BC044933 | Homo sapiens, clone IMAGE:4540326, mRNA, partial cds | 5.46 Up | 2.16E-04 |
| NM_001353 | Homo sapiens aldo-keto reductase family 1, member C1 (dihydrodiol | 5.46 Up | 4.58E-05 |
| | dehydrogenase 1; 20-alpha (3-alpha)-hydroxysteroid dehydrogenase) (AKR1C1), mRNA | | |
| NM_145061 | Homo sapiens chromosome 13 open reading frame 3 (C13orf3), mRNA | 5.41 Up | 1.05E-04 |
| AF131784 | Homo sapiens clone 25194 mRNA sequence | 5.41 Up | 2.51E-04 |
| NM_152515 | Homo sapiens hypothetical protein FLJ40629 (FLJ40629), mRNA | 5.36 Up | 4.28E-04 |
| NM_021000 | Homo sapiens pituitary tumor-transforming 3 (PTTG3), mRNA | 5.34 Up | 3.12E-04 |
| NM_003318 | Homo sapiens TTK protein kinase (TTK), mRNA | 5.34 Up | 2.64E-04 |
| NM_003259 | Homo sapiens intercellular adhesion molecule 5, telencephalin (ICAM5), mRNA | 5.33 Up | 8.55E-04 |
| NM_199414 | Homo sapiens protein regulator of cytokinesis 1 (PRC1), transcript variant 3, mRNA | 5.32 Up | 2.24E-05 |
| NM_006845 | Homo sapiens kinesin family member 2C (KIF2C), mRNA | 5.28 Up | 2.09E-04 |
| NM_032849 | Homo sapiens hypothetical protein FLJ14834 (FLJ14834), mRNA | 5.28 Up | 3.44E-04 |
| NM_006101 | Homo sapiens kinetochore associated 2 (KNTC2), mRNA | 5.25 Up | 1.22E-04 |
| AA662240 | nu89c01.s1 NCI_CGAP_AIv1 Homo sapiens cDNA clone IMAGE:1217856, mRNA sequence | 5.24 Up | 7.33E-04 |
| NM_001826 | Homo sapiens CDC28 protein kinase regulatory subunit 1B (CKS1B), mRNA | 5.21 Up | 2.51E-04 |
| NM_024745 | Homo sapiens SHC SH2-domain binding protein 1 (SHCBP1), mRNA | 5.16 Up | 4.90E-04 |
| NM_016426 | Homo sapiens G-2 and S-phase expressed 1 (GTSE1), mRNA | 5.16 Up | 4.20E-03 |
| NM_001813 | Homo sapiens centromere protein E, 312kDa (CENPE), mRNA | 5.15 Up | 3.41E-06 |
| NM_031966 | Homo sapiens cyclin B1 (CCNB1), mRNA | 5.13 Up | 4.25E-05 |
| BE735115 | 601566084F1 NIH_MGC_21 Homo sapiens cDNA clone IMAGE:3840837 5, mRNA sequence | 5.05 Up | 2.74E-04 |
| BG939678 | cr60d11.x1 Human bone marrow stromal cells Homo sapiens cDNA clone HBMSC_cr60d11 3, mRNA sequence | 5 Up | 1.62E-03 |
| NM_002474 | Homo sapiens myosin, heavy polypeptide 11, smooth muscle (MYH11), transcript variant SM1, mRNA | 95.15 Down | 2.77E-05 |
| BG571477 | 602592765F1 NIH_MGC_79 Homo sapiens cDNA clone IMAGE:4720025 5, | 13.02 Down | 6.90E-04 |
| | mRNA sequence | | |
| NM_002986 | Homo sapiens chemokine (C-C motif) ligand 11 (CCL11), mRNA | 12.42 Down | 3.33E-04 |
| AL713608 | DKFZp6860179_r1 686 (synonym: hlcc3) Homo sapiens cDNA clone DKFZp6860179 5, mRNA sequence | 11.81 Down | 4.28E-04 |
| NM_153267 | Homo sapiens MAM domain containing 2 (MAMDC2), mRNA | 10.38 Down | 3.46E-05 |
| W92068 | zh48g03.r1 Soares_fetal_liver_spleen_1NFLS_S1 Homo sapiens cDNA clone IMAGE:415348 5, mRNA sequence | 9.47 Down | 2.37E-04 |
| NM_017680 | Homo sapiens asporin (LRR class 1) (ASPN), mRNA | 8.99 Down | 4.58E-05 |
| AA608841 | af83g04.s1 Soares_testis_NHT Homo sapiens cDNA clone IMAGE:1048662 3, mRNA sequence | 8.59 Down | 4.37E-04 |
| AL134451 | DKFZp547J015_r1 547 (synonym: hfbr1) Homo sapiens cDNA clone DKFZp547J015 5, mRNA sequence | 8.53 Down | 2.51E-04 |
| NM_006774 | Homo sapiens indolethylamine N-methyltransferase (INMT), mRNA | 8.49 Down | 1.07E-04 |
| NM_006774 | Homo sapiens indolethylamine N-methyltransferase (INMT), mRNA | 8.35 Down | 2.51E-04 |
| AK124751 | Homo sapiens cDNA FLJ42761 fis, clone BRAWH3002574, highly similar to Calpain 2, large [catalytic] subunit precursor (EC 3.4.22.17) | 8.22 Down | 9.94E-05 |
| NM_001001430 | Homo sapiens troponin T2, cardiac (TNNT2), transcript variant 2, mRNA | 8.1 Down | 4.25E-05 |
| BX119527 | BX119527 Soares_fetal_liver_spleen_1 NFLS_S1 Homo sapiens cDNA clone IMAGp998O214529 ; IMAGE:1851116, mRNA sequence | 7.44 Down | 2.88E-04 |
| BF940114 | nac68c06.x1 NCI_CGAP_Brn23 Homo sapiens cDNA clone IMAGE:3439475 3, mRNA sequence | 7.4 Down | 6.95E-04 |
| AI221408 | qg92c01.x1 Soares_NFL_T_GBC_S1 Homo sapiens cDNA clone IMAGE:1842624 3, mRNA sequence | 7.03 Down | 6.90E-04 |
| NM_012278 | Homo sapiens integrin beta 1 binding protein (melusin) 2 (ITGB1BP2), mRNA | 6.89 Down | 3.76E-04 |
| NM_025202 | Homo sapiens EF hand domain containing 1 (EFHD1), mRNA | 6.87 Down | 4.71 E-05 |
| NM_000961 | Homo sapiens prostaglandin 12 (prostacyclin) synthase (PTGIS), mRNA | 6.87 Down | 3.96E-04 |
| D29134 | HUMNK158 Human epidermal | 6.86 Down | 4.28E-04 |
| | keratinocyte Homo sapiens cDNA clone 158, mRNA sequence | | |
| BX107738 | BX107738 Soares_testis_NHT Homo sapiens cDNA clone IMAGp998F181825 ; IMAGE:743057, mRNA sequence | 6.71 Down | 1.65E-04 |
| NM_006587 | Homo sapiens corin, serine protease (CORIN), mRNA | 6.64 Down | 9.90E-04 |
| AF070632 | Homo sapiens clone 24405 mRNA sequence | 6.47 Down | 9.94E-05 |
| NM_032762 | Homo sapiens hypothetical protein MGC16121 (MGC16121), mRNA | 6.44 Down | 6.95E-04 |
| NM_002587 | Homo sapiens protocadherin 1 (cadherin-like 1) (PCDH1), transcript variant 1, mRNA | 6.39 Down | 2.77E-05 |
| AV700621 | AV700621 GKC Homo sapiens cDNA clone GKCDKF09 3, mRNA sequence | 6.33 Down | 2.84E-03 |
| R67051 | yi30b07.s1 Soares placenta Nb2HP Homo sapiens cDNA clone IMAGE:140725 3, mRNA sequence | 6.29 Down | 6.14E-04 |
| NM_052890 | Homo sapiens peptidoglycan recognition protein 2 (PGLYRP2), mRNA | 6.21 Down | 6.86E-05 |
| AF220263 | Homo sapiens MOST2 mRNA, complete cds | 6.18 Down | 1.68E-04 |
| NM_014476 | Homo sapiens PDZ and LIM domain 3 (PDLIM3), mRNA | 6.04 Down | 4.17E-04 |
| NM_000095 | Homo sapiens cartilage oligomeric matrix protein (COMP), mRNA | 5.96 Down | 4.11E-03 |
| AI954275 | wx95c09.x1 NCI_CGAP_Mel15 Homo sapiens cDNA clone IMAGE:2551408 3, mRNA sequence | 5.95 Down | 1.15E-03 |
| NM_005202 | Homo sapiens collagen, type VIII, alpha 2 (COL8A2), mRNA | 5.76 Down | 2.77E-05 |
| BX649033 | Homo sapiens mRNA; cDNA DKFZp686K1098 (from clone DKFZp686K1098) | 5.73 Down | 3.96E-04 |
| AI392805 | tg04h03.x1 NCI_CGAP_CLL1 Homo sapiens cDNA clone IMAGE:2107829 3, mRNA sequence | 5.63 Down | 3.84E-04 |
| NM_002217 | Homo sapiens inter-alpha (globulin) inhibitor H3 (ITIH3), mRNA | 5.54 Down | 4.28E-04 |
| CA773752 | im56f03.y1 HR85 islet Homo sapiens cDNA clone IMAGE:6039292 5, mRNA sequence | 5.48 Down | 1.62E-03 |
| NM_003725 | Homo sapiens 3-hydroxysteroid epimerase (RODH), mRNA | 5.45 Down | 3.96E-04 |
| T71557 | yd36b08.s1 Soares fetal liver spleen 1NFLS Homo sapiens cDNA clone IMAGE:110295 3, mRNA sequence | 5.39 Down | 4.17E-04 |
| AW297946 | UI-H-BWO-ajn-d-08-0-UI.s1 NCI_CGAP_Sub6 Homo sapiens cDNA clone IMAGE:2732223 3, mRNA sequence | 5.39 Down | 2.50E-03 |
| AK025101 | Homo sapiens cDNA: FLJ21448 fis, clone COL04473 | 5.38 Down | 2.87E-03 |
| NM_005725 | Homo sapiens tetraspan 2 (TSPAN-2), mRNA | 5.37 Down | 1.24E-03 |
| AW 136248 | UI-H-BI1-act-e-05-0-UI.s1 NCI_CGAP_Sub3 Homo sapiens cDNA clone IMAGE:2715369 3, mRNA sequence | 5.37 Down | 1.11E-03 |
| NM_001854 | Homo sapiens collagen, type XI, alpha 1 (COL11A1), transcript variant A, mRNA | 5.35 Down | 4.42E-04 |
| AW075298 | xa92c06.x1 NCI_CGAP_Co17 Homo sapiens cDNA clone IMAGE:2574250 3, mRNA sequence | 5.33 Down | 6.95E-04 |
| AA903192 | ok48d01.s1 NCI_CGAP_Lei2 Homo sapiens cDNA clone IMAGE:1517185 3, mRNA sequence | 5.29 Down | 2.97E-03 |
| AV700217 | AV700217 GKC Homo sapiens cDNA clone GKCDNE03 3, mRNA sequence | 5.26 Down | 2.31 E-03 |
| AA633962 | ac73h01.s1 Stratagene lung (#937210) Homo sapiens cDNA clone IMAGE:868273 3 similar to gb:M57627 INTERLEUKIN-10 PRECURSOR (HUMAN);contains element MER16 repetitive element ;, mRNA sequence | 5.26 Down | 9.43E-04 |
| BQ439091 | AGENCOURT-7761579 NIH_MGC_70 Homo sapiens cDNA clone IMAGE:6020085 5, mRNA sequence | 5.26 Down | 3.33E-04 |
| H41942 | yo60a08.r1 Soares breast 3NbHBst Homo sapiens cDNA clone IMAGE:182294 5 similar to contains Alu repetitive element;contains LTR9 repetitive element RNA sequence | 5.25 Down | 9.94E-05 |
| AK021531 | Homo sapiens cDNA FLJ11469 fis, clone HEMBA1001658 | 5.2 Down | 1.04E-03 |
| AA558704 | nI42c03.s1 NCI_CGAP_Pr4 Homo sapiens cDNA clone IMAGE:1043332, mRNA sequence | 5.19 Down | 2.31E-03 |
| AA846538 | aj56f12.s1 Soares_testis_NHT Homo sapiens cDNA clone IMAGE:1394351 3, mRNA sequence | 5.19 Down | 4.16E-04 |
| AI766299 | wh71c10.x1 NCI_CGAP_Kid11 Homo sapiens cDNA clone IMAGE:2386194 3, mRNA sequence | 5.14 Down | 4.48E-04 |
| BE173027 | MR0-HT0559-200400-015-e07_1 | 5.11 Down | 1.04E-03 |
| | HT0559 Homo sapiens cDNA, mRNA sequence | | |
| BE697175 | RC1-CT0414-260700-011-a03 CT0414 Homo sapiens cDNA, mRNA sequence | 5.1 Down | 4.42E-04 |
| AI805522 | tx86b02.x1 NCI_CGAP_Ut4 Homo sapiens cDNA clone IMAGE:2276427 3, mRNA sequence | 5.08 Down | 1.50E-03 |
| NM_003287 | Homo sapiens tumor protein D52-like 1 (TPD52L1), transcript variant 1, mRNA | 5.06 Down | 5.00E-04 |
| CA948483 | iq27c09.x1 HR85 islet Homo sapiens cDNA clone IMAGE: 3, mRNA sequence | 5.05 Down | 1.38E-03 |

**TABLE XVI: GENES THAT WERE DIFFERENTIALLY EXPRESSED AT LEAST 5-FOLD BETWEEN ADULT PANCREATIC-DERIVED STROMAL CELLS DERIVED FROM THE F4 AND F3 FRACTIONS OF A SINGLE DONOR HUMAN PANCREAS.**

| Gene Identifier | Gene Name | Ratio (F4/F3) Direction | adj. p-value |
|---|---|---|---|
| NM_000961 | Homo sapiens prostaglandin 12 (prostacyclin) synthase (PTGIS), mRNA | 19.44 Up | 7.06E-05 |
| NM_198389 | Homo sapiens lung type-I cell membrane-associated glycoprotein (T1A-2), transcript variant 2, mRNA | 12.97 Up | 1.01E-04 |
| NM_018894 | Homo sapiens EGF-containing fibulin-like extracellular matrix protein 1 (EFEMP1), transcript variant 2, mRNA | 10.65 Up | 7.32E-04 |
| NM_014178 | Homo sapiens syntaxin binding protein 6 (amisyn) (STXBP6), mRNA | 10.23 Up | 5.37E-05 |
| BE904671 | 601498784F1 NIH_MGC_70 Homo sapiens cDNA clone IMAGE:3900717 5, mRNA sequence | 8.72 Up | 6.44E-04 |
| NM_003014 | Homo sapiens secreted frizzled-related protein 4 (SFRP4), mRNA | 8.64 Up | 3.28E-04 |
| NM_001998 | Homo sapiens fibulin 2 (FBLN2), transcript variant 2, mRNA | 8.1 Up | 7.69E-03 |
| R41565 | yf88e01.s1 Soares infant brain 1NIB Homo sapiens cDNA clone IMAGE:29531 3, mRNA sequence | 7.68 Up | 8.31E-05 |
| NM_153256 | Homo sapiens chromosome 10 open reading frame 47 (C10orf47), mRNA | 7.16 Up | 5.46E-04 |
| AF010236 | Homo sapiens mRNA from chromosome 5q31-33 region | 7.12 Up | 3.72E-04 |
| AB058769 | Homo sapiens mRNA for KIAA1866 protein, partial cds | 7.04 Up | 2.63E-04 |
| NM_021012 | Homo sapiens potassium inwardly-rectifying channel, subfamily J, member 12 (KCNJ12), mRNA | 7.02 Up | 1.09E-03 |
| D29453 | HUMNK566 Human epidermal keratinocyte Homo sapiens cDNA clone 566, mRNA sequence | 7.02 Up | 1.53E-04 |
| BX648964 | Homo sapiens mRNA; cDNA DKFZp686J0156 (from clone DKFZp686J0156) | 6.86 Up | 8.31E-05 |
| NM_017805 | Homo sapiens Ras interacting protein 1 (RASIP1), mRNA | 6.56 Up | 6.43E-04 |
| CA428652 | UI-H-FH1-bfe-k-11-0-UI.s1 | 6.41 Up | 1.01E-04 |
| | NCI_CGAP_FH1 Homo sapiens cDNA clone UI-H-FH1-bfe-k-11-0-UI 3, mRNA sequence | | |
| NM_005213 | Homo sapiens cystatin A (stefin A) (CSTA), mRNA | 6.24 Up | 3.33E-05 |
| NM_005127 | Homo sapiens C-type (calcium dependent, carbohydrate-recognition domain) lectin, superfamily member 2 (activation-induced) (CLECSF2), mRNA | 6.17 Up | 1.10E-04 |
| NM_006183 | Homo sapiens neurotensin (NTS), mRNA | 5.98 Up | 7.13E-04 |
| AB011538 | Homo sapiens mRNA for MEGF5, partial cds | 5.93 Up | 9.44E-05 |
| AA813769 | ai69h12.s1 Soares_testis_NHT Homo sapiens cDNA clone 1376135 3, mRNA sequence | 5.78 Up | 1.10E-04 |
| NM_001276 | Homo sapiens chitinase 3-like 1 (cartilage glycoprotein-39) (CHI3L1), mRNA | 5.52 Up | 3.33E-05 |
| AF070632 | Homo sapiens clone 24405 mRNA sequence | 5.47 Up | 7.80E-05 |
| NM_005807 | Homo sapiens proteoglycan 4 (PRG4), mRNA | 5.34 Up | 3.05E-03 |
| NM_002474 | Homo sapiens myosin, heavy polypeptide 11, smooth muscle (MYH11), transcript variant SM1, mRNA | 5.27 Up | 1.01E-04 |
| BQ435580 | AGENCOURT_7836890 NIH_MGC_82 Homo sapiens cDNA clone IMAGE:6102371 5, mRNA sequence | 5.24 Up | 8.31E-05 |
| NM_000104 | Homo sapiens cytochrome P450, family 1, subfamily B, polypeptide 1 (CYP1B1), mRNA | 5.23 Up | 1.56E-04 |
| AW294090 | UI-H-BI2-ahg-b-12-0-UI.s1 NCI_CGAP_Sub4 Homo sapiens cDNA clone IMAGE:2726734 3, mRNA sequence | 5.2 Up | 6.44E-04 |
| NM_001928 | Homo sapiens D component of complement (adipsin) (DF), mRNA | 5.08 Up | 9.52E-04 |
| NM_002245 | Homo sapiens potassium channel, subfamily K, member 1 (KCNK1), mRNA | 5.05 Up | 5.46E-04 |

Although the various aspects of the invention have been illustrated above by reference to examples and preferred embodiments, it will be appreciated that the scope of the invention is defined not by the foregoing description, but by the following claims properly construed under principles of patent law.

## Claims

1. A population of adult pancreatic-derived stromal cells, wherein the cells are negative for the expression of NCAM, ABCG2, cytokeratin 7, cytokeratin 8, cytokeratin 18, and cytokeratin 19, and wherein the cells of the population are positive for the expression of at least one protein marker selected from the group consisting of CD44, CD73, CD90 or CD105.

2. The population of adult pancreatic-derived stromal cells according to claim 1, capable of propagating *in vitro.*

3. The population of adult pancreatic-derived stromal cells according to claim 2, capable of propagating under hypoxic conditions.

4. The population of adult pancreatic-derived stromal cells according to claim 1, capable of differentiating into cells displaying the characteristics of the β-cell lineage.

5. The population of adult pancreatic-derived stromal cells according to claim 1 or 2 that has been genetically engineered to over express a protein.

6. The population of adult pancreatic-derived stromal cells according to claim 1 or 2 that has been genetically engineered to decrease the expression of a protein.

7. A method of screening for agents capable of inducing adult pancreatic-derived stromal cells to differentiate into a cell of a β-cell lineage using the cells of claim 1, 5 or 6 comprising:
a. Culturing the adult pancreatic-derived stromal cells of claim 1, 5 or 6,
b. Determining the expression levels of pancreatic markers in the adult pancreatic-derived stromal cells,
c. Contacting and incubating the adult pancreatic-derived stromal cells with an agent capable of inducing cells to differentiate into a cell of the β-cell lineage,
d. Determining the expression levels of pancreatic markers in the adult pancreatic-derived stromal cells, and
e. Monitoring changes in gene expression over time.

8. A method of screening for agents capable of altering the expression of a gene or a protein in the adult pancreatic-derived stromal cells of claim 1, 5 or 6, comprising:
a. Culturing the adult pancreatic-derived stromal cells of claim 1, 5 or 6,
b. Determining the expression levels of a gene or a protein in the adult pancreatic-derived stromal cells,
c. Contacting and incubating the adult pancreatic-derived stromal cells with an agent capable of altering the expression of said gene or said protein,
d. Determining the expression levels of said gene or said protein in the adult pancreatic-derived stromal cells, and
e. Monitoring changes in gene expression over time.

9. An *in vitro* method of obtaining a population of cells enriched in stromal cells from a human pancreas, wherein the cells are negative for the expression of NCAM, ABCG2, cytokeratin 7, cytokeratin 8, cytokeratin 18, and cytokeratin 19, comprising:
a. Culturing isolated pancreatic cells from the human pancreas in a basal nutrient medium supplemented with serum and glucose at a concentration of below about 30mM; and
b. Allowing the cells to grow in the medium for about two weeks, thereby obtaining a population of cells enriched in stromal cells;
and wherein the cells of the population are positive for the expression of at least one protein marker selected from the group consisting of CD44, CD73, CD90 or CD105.

10. The method of claim 9, wherein the stromal cells in the population of cells are capable of propagating *in vitro.*

11. The method of claim 9, wherein the stromal cells in the population of cells are capable of differentiating into cells displaying the characteristics of the β-cell lineage.

12. The method of claim 9, further comprising:
a. Selecting and isolating the stromal cells based on at least one protein marker selected from the group consisting of CD44, CD73, CD90 or CD105.

13. The method of claim 9, further comprising a step (d) of isolating cells that specifically bind to an agent specific for a protein marker specifically expressed in the adult pancreatic-derived stromal cells, thereby obtaining a pure population of adult pancreatic-derived stromal cells.

14. The method of claim 13, wherein the protein marker is selected from the group consisting of CD44, CD73, CD90 or CD105.

15. The method of claim 13, wherein the population of cells are negative for the expression of NCAM, ABCG2, cytokeratin 7, cytokeratin 8, cytokeratin 18, and cytokeratin 19.

16. The method of claim 13, wherein the population of cells are capable of propagating *in vitro.*

17. The method of claim 13, wherein the population of cells are capable of propagating under hypoxic conditions.

18. The method of claim 13, wherein the population of cells are capable of differentiating into cells displaying the characteristics of the β-cell lineage.

19. An *in vitro* method of obtaining cells displaying the characteristics of the β-cell lineage, comprising obtaining a pure population of adult pancreatic-derived stromal cells obtained from a human pancreas, and treating adult pancreatic-derived stromal cells of any one of claims 1-6 with an effective amount of an agent(s) that induces the adult pancreatic-derived stromal cells to differentiate into cells displaying the characteristics of the β-cell lineage.

20. The *in vitro* method of claim 19, wherein the population of adult pancreatic-derived stromal cells are obtained by the method, comprising:
a. Culturing isolated pancreatic cells from a human pancreas in a basal nutrient medium supplemented with serum and glucose at a concentration of below about 30mM;
b. Allowing the cells to grow in the medium for about two weeks to obtain a population of cells enriched in stromal cells; and
c. Isolating cells that specifically bind to an agent specific for a protein marker specifically expressed in the adult pancreatic-derived stromal cells, thereby obtaining a pure population of adult pancreatic-derived stromal cells.

21. The method of claim 12, 17 or 20, wherein the serum concentration is below about 5%.

22. The method of claim 19, wherein the population of adult pancreatic-derived stromal cells from a pancreas are expanded in culture prior to the treatment to induce differentiation.

23. The method of claim 19, wherein the cells displaying the characteristics of the β-cell lineage are insulin-producing cells.

24. The adult pancreatic-derived stromal cells of any one of claims 1-6 for use in the treatment of a patient with diabetes mellitus or at risk of developing diabetes, wherein the stromal cells are obtained by isolating a population of adult pancreatic-derived stromal cells from donor pancreatic tissue, and wherein upon transferring the adult pancreatic-derived stromal cells into the patient, the adult pancreatic-derived stromal cells differentiate into pancreatic hormone producing cells.

25. Progenitor cells for use in the treatment of a patient with diabetes mellitus or at risk of developing diabetes, wherein the progenitor cells are obtained by an *ex vivo* method comprising:
expanding *ex vivo* stromal cells that have been isolated from donor pancreatic tissue wherein the cells are negative for the expression of NCAM, ABCG2, cytokeratin 7, cytokeratin 8, cytokeratin 18, and
cytokeratin 19, and wherein the cells of the population are positive for the expression of at least one protein marker selected from the group consisting of CD44, CD73, CD90 or CD105, to produce progenitor cells, and wherein upon transferring the progenitor cells into the patient, the progenitor cells differentiate into pancreatic hormone producing cells.

26. The cells for use according to claim 24 or 25, wherein the pancreatic hormone producing cells are insulin-producing cells.

27. Cells of a pancreatic β-cell lineage for use in the treatment of a patient with diabetes mellitus or at risk of developing diabetes, wherein the cells of a pancreatic β-cell lineage are obtained by an *in vitro* method comprising:
(a) expanding *in vitro* a population of adult pancreatic-derived stromal cells that have been isolated from donor pancreatic tissue wherein the cells are negative for the expression of NCAM, ABCG2, cytokeratin 7, cytokeratin 8, cytokeratin 18, and cytokeratin 19, and wherein the cells of the population are positive for the expression of at least one protein marker selected from the group consisting of CD44, CD73, CD90 or CD105; and
(b) differentiating the expanded adult pancreatic-derived stromal cells into a pancreatic β cell lineage.

28. The cells for use according to claim 27, wherein the pancreatic β cell lineage cells are insulin-producing cells.

29. The cells for use according to claim 25, 26 or 27, wherein the patient is the donor of the pancreatic tissue.

30. The adult pancreatic-derived stromal cells and islets of any one of claims 1-6 for use in treating a patient with diabetes mellitus or at risk of developing diabetes, wherein the stromal cells and islets are obtained by a method comprising:
(a) isolating a population of adult pancreatic-derived stromal cells from a donor pancreatic tissue; and
(b) obtaining mature islets from a donor, and wherein upon transferring both the adult pancreatic-derived stromal cells and the islets into the patient:
(i) the islets enhance the survival and function of the adult pancreatic-derived stromal cells in the patient; or
(ii) the adult pancreatic-derived stromal cells enhance the survival and function of the islets in the patient.

31. The cells for use according to claim 30, wherein the donor of the mature islets is allogeneic or xenogeneic relative to the patient.

32. The cells for use according to any one of claims 24, 25, 27 or 30, wherein the cells to be transferred to the patient are incorporated in a polymeric support material.

33. The cells for use according to claim 32, wherein the polymeric support material is also loaded with a pharmaceutical carrier or bioactive agent that facilitates the survival and function of the cells in the patient.

## Patentansprüche

1. Population von vom Pankreas Erwachsener abgeleiteten Stromazellen, wobei die Zellen für die Expression von NCAM, ABCG2, Cytokeratin 7, Cytokeratin 8, Cytokeratin 18 und Cytokeratin 19 negativ sind und wobei die Zellen der Population für die Expression von mindestens einem Protein-marker, ausgewählt aus der Gruppe bestehend aus CD44, CD73, CD90 oder CD105, positiv sind.

2. Population von vom Pankreas Erwachsener abgeleiteten Stromazellen nach Anspruch 1, die zur *invitro*-Vermehrung fähig ist.

3. Population von vom Pankreas Erwachsener abgeleiteten Stromazellen nach Anspruch 2, die zur Vermehrung unter Sauerstoffmangelbedingungen fähig ist.

4. Population von vom Pankreas Erwachsener abgeleiteten Stromazellen nach Anspruch 1, die fähig ist, sich zu Zellen, die die Merkmale der β-Zelllinie aufweisen, zu differenzieren.

5. Population von vom Pankreas Erwachsener abgeleiteten Stromazellen nach Anspruch 1 oder 2, die genetisch so verändert worden ist, dass sie ein Protein überexprimiert.

6. Population von vom Pankreas Erwachsener abgeleiteten Stromazellen nach Anspruch 1 oder 2, die genetisch so verändert worden ist, dass sie die Expression eines Proteins vermindert.

7. Verfahren zum Screening von Agenzien, die fähig sind, vom Pankreas Erwachsener abgeleitete Stromazellen dazu zu induzieren, sich zu einer Zelle einer β-Zelllinie zu differenzieren, und zwar unter Verwendung der Zellen nach Anspruch 1, 5 oder 6, wobei das Verfahren Folgendes umfasst:
a. Kultivieren der vom Pankreas Erwachsener abgeleiteten Stromazellen nach Anspruch 1, 5 oder 6,
b. Bestimmung der Expressionsniveaus von Pankreasmarkern in den vom Pankreas Erwachsener abgeleiteten Stromazellen,
c. Inkontaktbringen und Inkubieren der vom Pankreas Erwachsener abgeleiteten Stromazellen mit einem Agens, das fähig ist, die Zellen dazu zu induzieren, sich zu einer Zelle der β-Zelllinie zu differenzieren,
d. Bestimmung der Expressionsniveaus von Pankreasmarkern in den vom Pankreas Erwachsener abgeleiteten Stromazellen, und
e. Beobachten von Veränderungen der Genexpression als Funktion der Zeit.

8. Verfahren zum Screening von Agenzien, die fähig sind, die Expression eines Gens oder eines Proteins in den vom Pankreas Erwachsener abgeleiteten Stromazellen nach Anspruch 1, 5 oder 6 zu verändern, wobei das Verfahren Folgendes umfasst:
a. Kultivieren der vom Pankreas Erwachsener abgeleiteten Stromazellen nach Anspruch 1, 5 oder 6,
b. Bestimmung der Expressionsniveaus eines Gens oder eines Proteins in den vom Pankreas Erwachsener abgeleiteten Stromazellen,
c. Inkontaktbringen und Inkubieren der vom Pankreas Erwachsener abgeleiteten Stromazellen mit einem Agens, das fähig ist, die Expression des Gens oder des Proteins zu verändern,
d. Bestimmung der Expressionsniveaus des Gens oder des Proteins in den vom Pankreas Erwachsener abgeleiteten Stromazellen, und
e. Beobachten von Veränderungen der Genexpression als Funktion der Zeit.

9. *In-vitro*-Verfahren zum Erhalten einer Population von Zellen, die an Stromazellen aus einem menschlichen Pankreas angereichert ist, wobei die Zellen für die Expression von NCAM, ABCG2, Cytokeratin 7, Cytokeratin 8, Cytokeratin 18 und Cytokeratin 19 negativ sind, wobei das Verfahren Folgendes umfasst:
a. Kultivieren von isolierten Pankreaszellen aus dem menschlichen Pankreas in einem Basalnährmedium mit einem Zusatz von Serum und Glucose in einer Konzentration von unter ungefähr 30 mM; und
b. Wachsenlassen der Zellen in dem Medium für ungefähr zwei Wochen, wodurch man eine Population von Zellen, die an Stromazellen angereichert ist, erhält;
und wobei die Zellen der Population für die Expression von mindestens einem Proteinmarker, ausgewählt aus der Gruppe bestehend aus CD44, CD73, CD90 oder CD105, positiv sind.

10. Verfahren nach Anspruch 9, wobei die Stromazellen in der Population von Zellen zur *in-vitro-*Vermehrung fähig sind.

11. Verfahren nach Anspruch 9, wobei die Stromazellen in der Population von Zellen fähig sind, sich zu Zellen, die die Merkmale der β-Zelllinie aufweisen, zu differenzieren.

12. Verfahren nach Anspruch 9, das weiterhin Folgendes umfasst:
a. Selektieren und Isolieren der Stromazellen aufgrund mindestens eines Proteinmarkers, ausgewählt aus der Gruppe bestehend aus CD44, CD73, CD90 oder CD105.

13. Verfahren nach Anspruch 9, das weiterhin einen Schritt (d) umfasst: Isolieren von Zellen, die spezifisch an ein Agens mit Spezifität für einen Proteinmarker, der spezifisch in den vom Pankreas Erwachsener abgeleiteten Stromazellen exprimiert wird, binden, wodurch man zu einer reinen Population von vom Pankreas Erwachsener abgeleiteten Stromazellen gelangt.

14. Verfahren nach Anspruch 13, wobei der Protein-marker aus der Gruppe bestehend aus CD44, CD73, CD90 oder CD105 ausgewählt ist.

15. Verfahren nach Anspruch 13, wobei die Population von Zellen für die Expression von NCAM, ABCG2, Cytokeratin 7, Cytokeratin 8, Cytokeratin 18 und Cytokeratin 19 negativ ist.

16. Verfahren nach Anspruch 13, wobei die Population von Zellen zur *in-vitro*-Vermehrung fähig ist.

17. Verfahren nach Anspruch 13, wobei die Population von Zellen zur Vermehrung unter Sauerstoffmangelbedingungen fähig ist.

18. Verfahren nach Anspruch 13, wobei die Population von Zellen fähig ist, sich zu Zellen, die die Merkmale der β-Zelllinie aufweisen, zu differenzieren.

19. *In-vitro*-Verfahren zum Erhalten von Zellen, die die Merkmale der β-Zelllinie aufweisen, umfassend das Erhalten einer reinen Population von vom Pankreas Erwachsener abgeleiteten Stromazellen, die von einem menschlichen Pankreas stammen, und Behandeln von vom Pankreas Erwachsener abgeleiteten Stromazellen nach einem der Ansprüche 1-6 mit einer wirksamen Menge eines Agens bzw. von Agenzien, das bzw. die die vom Pankreas Erwachsener abgeleiteten Stromazellen dazu induziert bzw. induzieren, sich zu Zellen, die die Merkmale der β-Zelllinie aufweisen, zu differenzieren.

20. *In-vitro*-Verfahren nach Anspruch 19, wobei die Population von vom Pankreas Erwachsener abgeleiteten Stromazellen durch ein Verfahren erhalten wird, das Folgendes umfasst:
a. Kultivieren von isolierten Pankreaszellen aus einem menschlichen Pankreas in einem Basalnährmedium mit einem Zusatz von Serum und Glucose in einer Konzentration von unter ungefähr 30 mM;
b. Wachsenlassen der Zellen in dem Medium für ungefähr zwei Wochen, wodurch man eine Population von Zellen, die an Stromazellen angereichert ist, erhält; und
c. Isolieren von Zellen, die spezifisch an ein Agens mit Spezifität für einen Proteinmarker, der spezifisch in den vom Pankreas Erwachsener abgeleiteten Stromazellen exprimiert wird, binden, wodurch man zu einer reinen Population von vom Pankreas Erwachsener abgeleiteten Stromazellen gelangt.

21. Verfahren nach Anspruch 12, 17 oder 20, wobei die Serumkonzentration unter ungefähr 5% liegt.

22. Verfahren nach Anspruch 19, wobei die Population von vom Pankreas Erwachsener abgeleiteten Stromazellen aus einem Pankreas vor der Behandlung zur Induktion von Differenzierung in Kultur expandiert wird.

23. Verfahren nach Anspruch 19, wobei es sich bei den Zellen, die die Merkmale der β-Zelllinie aufweisen, um insulinproduzierende Zellen handelt.

24. Vom Pankreas Erwachsener abgeleitete Stromazellen nach einem der Ansprüche 1-6 zur Verwendung in der Behandlung eines Patienten mit Diabetes mellitus oder eines Patienten, bei dem die Gefahr des Auftretens von Diabetes besteht, wobei die Stromazellen durch Isolieren einer Population von vom Pankreas Erwachsener abgeleiteten Stromazellen aus Donorpankreasgewebe erhalten werden, und wobei sich beim Einbringen der vom Pankreas Erwachsener abgeleiteten Stromzellen in den Patienten die vom Pankreas Erwachsener abgeleiteten Stromazellen zu pankreashormonproduzierenden Zellen differenzieren.

25. Vorläuferzellen zur Verwendung in der Behandlung eines Patienten mit Diabetes mellitus oder eines Patienten, bei dem die Gefahr des Auftretens von Diabetes besteht, wobei die Vorläuferzellen durch ein *ex-vivo*-Verfahren erhalten werden, das Folgendes umfasst:
*ex-vivo*-Expandieren von Stromazellen, die aus Donorpankreasgewebe isoliert worden sind, wobei die Zellen für die Expression von NCAM, ABCG2, Cytokeratin 7, Cytokeratin 8, Cytokeratin 18 und Cytokeratin 19 negativ sind und wobei die Zellen der Population für die Expression von mindestens einem Proteinmarker, ausgewählt aus der Gruppe bestehend aus CD44, CD73, CD90 oder CD105, positiv sind, um Vorläuferzellen zu erzeugen, und wobei sich beim Einbringen der Vorläuferzellen in den Patienten die Vorläuferzellen zu pankreashormonproduzierenden Zellen differenzieren.

26. Zellen zur Verwendung nach Anspruch 24 oder 25, wobei es sich bei den pankreashormonproduzierenden Zellen um insulinproduzierende Zellen handelt.

27. Zellen einer Pankreas-β-Zelllinie zur Verwendung in der Behandlung eines Patienten mit Diabetes mellitus oder eines Patienten, bei dem die Gefahr des Auftretens von Diabetes besteht, wobei die Zellen einer Pankreas-β-Zelllinie durch ein *in-vitro*-Verfahren erhalten werden, das Folgendes umfasst:
(a) *in-vitro*-Expandieren einer Population von vom Pankreas Erwachsener abgeleiteten Stromazellen, die aus Donorpankreasgewebe isoliert worden sind, wobei die Zellen für die Expression von NCAM, ABCG2, Cytokeratin 7, Cytokeratin 8, Cytokeratin 18 und Cytokeratin 19 negativ sind und wobei die Zellen der Population für die Expression von mindestens einem Proteinmarker, ausgewählt aus der Gruppe bestehend aus CD44, CD73, CD90 oder CD105, positiv sind; und
(b)Differenzieren der expandierten vom Pankreas Erwachsener abgeleiteten Stromazellen zu einer Pankreas-β-Zelllinie.

28. Zellen zur Verwendung nach Anspruch 27, wobei es sich bei den Pankreas-β-Zelllinie-Zellen um insulinproduzierende Zellen handelt.

29. Zellen zur Verwendung nach Anspruch 25, 26 oder 27, wobei der Patient der Donor des Pankreasgewebes ist.

30. Vom Pankreas Erwachsener abgeleitete Stromazellen und Inselzellen nach Anspruch 1-6 zur Verwendung in der Behandlung eines Patienten mit Diabetes mellitus oder eines Patienten, bei dem die Gefahr des Auftretens von Diabetes besteht, wobei die Stromazellen und Inselzellen nach einem Verfahren erhalten werden, das Folgendes umfasst:
(a)Isolieren einer Population von vom Pankreas Erwachsener abgeleiteten Stromazellen aus einem Donorpankreasgewebe; und
(b)Gewinnen von reifen Inselzellen von einem Donor, und wobei beim Einbringen sowohl der vom Pankreas Erwachsener abgeleiteten Stromazellen als auch der Inselzellen in den Patienten:
(i)die Inselzellen das Überleben und die Funktion der vom Pankreas Erwachsener abgeleiteten Stromazellen in dem Patienten erhöhen; oder
(ii)die vom Pankreas Erwachsener abgeleiteten Stromazellen das Überleben und die Funktion der Inselzellen in dem Patienten erhöhen.

31. Zellen zur Verwendung nach Anspruch 30, wobei der Donor der reifen Inselzellen allogen oder xenogen in Bezug auf den Patienten ist.

32. Zellen zur Verwendung nach einem der Ansprüche 24, 25, 27 oder 30, wobei die Zellen, die in den Patienten einzubringen sind, in ein polymeres Trägermaterial eingebaut sind.

33. Zellen zur Verwendung nach Anspruch 32, wobei das polymere Trägermaterial auch mit einem pharmazeutischen Träger oder biologisch aktiven Agens, der/das das Überleben und die Funktion der Zellen in dem Patienten fördert, beladen ist.

## Revendications

1. Population de cellules stromales dérivées de cellules pancréatiques adultes, dans laquelle les cellules sont négatives pour l'expression de NCAM, ABCG2, la cytokératine 7, la cytokératine 8, la cytokératine 18 et la cytokératine 19, et dans laquelle les cellules de la population sont positives pour l'expression d'au moins un marqueur protéique choisi dans le groupe constitué de CD44, CD73, CD90 ou CD105.

2. Population de cellules stromales dérivées de cellules pancréatiques adultes selon la revendication 1, capable de se multiplier *in vitro.*

3. Population de cellules stromales dérivées de cellules pancréatiques adultes selon la revendication 2, capable de se multiplier dans des conditions hypoxiques.

4. Population de cellules stromales dérivées de cellules pancréatiques adultes selon la revendication 1, capable de se différencier en cellules présentant les caractéristiques de la lignée de cellules β.

5. Population de cellules stromales dérivées de cellules pancréatiques adultes selon la revendication 1 ou 2, ayant été génétiquement modifiée pour sur-exprimer une protéine.

6. Population de cellules stromales dérivées de cellules pancréatiques adultes selon la revendication 1 ou 2, ayant été génétiquement modifiée pour présenter une diminution de l'expression d'une protéine.

7. Procédé de recherche par criblage d'agents capables d'induire la différenciation de cellules stromales dérivées de cellules pancréatiques adultes en une cellule d'une lignée de cellules β, utilisant les cellules selon la revendication 1, 5 ou 6, comprenant :
a. la culture des cellules stromales dérivées de cellules pancréatiques adultes selon la revendication 1, 5 ou 6,
b. la détermination des niveaux d'expression de marqueurs pancréatiques dans les cellules stromales dérivées de cellules pancréatiques adultes,
c. la mise en contact et l'incubation des cellules stromales dérivées de cellules pancréatiques adultes avec un agent capable d'induire la différenciation des cellules en une cellule de la lignée de cellules β,
d. la détermination des niveaux d'expression de marqueurs pancréatiques dans les cellules stromales dérivées de cellules pancréatiques adultes, et
e. le suivi des modifications de l'expression génique dans le temps.

8. Procédé de recherche par criblage d'agents capables de modifier l'expression d'un gène ou d'une protéine dans les cellules stromales dérivées de cellules pancréatiques adultes selon la revendication 1, 5 ou 6, comprenant :
a. la culture des cellules stromales dérivées de cellules pancréatiques adultes selon la revendication 1, 5 ou 6,
b. la détermination des niveaux d'expression d'un gène ou d'une protéine dans les cellules stromales dérivées de cellules pancréatiques adultes,
c. la mise en contact et l'incubation des cellules stromales dérivées de cellules pancréatiques adultes avec un agent capable de modifier l'expression dudit gène ou de ladite protéine,
d. la détermination des niveaux d'expression dudit gène ou de ladite protéine dans les cellules stromales dérivées de cellules pancréatiques adultes, et
e. le suivi des modifications de l'expression génique dans le temps.

9. Procédé *in vitro* d'obtention d'une population de cellules enrichie en cellules stromales provenant d'un pancréas humain, dans lequel les cellules sont négatives pour l'expression de NCAM, ABCG2, la cytokératine 7, la cytokératine 8, la cytokératine 18 et la cytokératine 19, comprenant :
a. la culture de cellules pancréatiques isolées provenant du pancréas humain dans un milieu nutritif basal additionné de sérum et de glucose à une concentration inférieure à environ 30 mM ; et
b. la prolifération des cellules dans le milieu pendant environ deux semaines, ce qui permet l'obtention d'une population de cellules enrichie en cellules stromales ;
et dans lequel les cellules de la population sont positives pour l'expression d'au moins un marqueur protéique choisi dans le groupe constitué de CD44, CD73, CD90 ou CD105.

10. Procédé selon la revendication 9, dans lequel les cellules stromales présentes dans la population de cellules sont capables de se multiplier *in vitro.*

11. Procédé selon la revendication 9, dans lequel les cellules stromales présentes dans la population de cellules sont capables de se différencier en cellules présentant les caractéristiques de la lignée de cellules β.

12. Procédé selon la revendication 9, comprenant, en outre :
a. la sélection et l'isolement des cellules stromales sur la base d'au moins un marqueur protéique choisi dans le groupe constitué de CD44, CD73, CD90 ou CD105.

13. Procédé selon la revendication 9, comprenant, en outre, une étape (d) d'isolement de cellules qui se lient, de façon spécifique, à un agent spécifique d'un marqueur protéique spécifiquement exprimé dans les cellules stromales dérivées de cellules pancréatiques adultes, ce qui permet l'obtention d'une population pure de cellules stromales dérivées de cellules pancréatiques adultes.

14. Procédé selon la revendication 13, dans lequel le marqueur protéique est choisi dans le groupe constitué de CD44, CD73, CD90 ou CD105.

15. Procédé selon la revendication 13, dans lequel la population de cellules est négative pour l'expression de NCAM, ABCG2, la cytokératine 7, la cytokératine 8, la cytokératine 18 et la cytokératine 19.

16. Procédé selon la revendication 13, dans lequel la population de cellules est capable de se multiplier *in vitro.*

17. Procédé selon la revendication 13, dans lequel la population de cellules est capable de se multiplier dans des conditions hypoxiques.

18. Procédé selon la revendication 13, dans lequel la population de cellules est capable de se différencier en cellules présentant les caractéristiques de la lignée des cellules β.

19. Procédé *in vitro* d'obtention de cellules présentant les caractéristiques de la lignée de cellules β, comprenant l'obtention d'une population pure de cellules stromales dérivées de cellules pancréatiques adultes obtenues à partir d'un pancréas humain, et le traitement des cellules stromales dérivées de cellules pancréatiques adultes selon l'une quelconque des revendications 1 à 6 à l'aide d'une quantité efficace d'au moins un agent induisant la différenciation des cellules stromales dérivées de cellules pancréatiques adultes en cellules présentant les caractéristiques de la lignée des cellules β.

20. Procédé *in vitro* selon la revendication 19, dans lequel la population de cellules stromales dérivées de cellules pancréatiques adultes est obtenue par le procédé, comprenant :
a. la culture de cellules pancréatiques isolées provenant d'un pancréas humain dans un milieu nutritif basal additionné de sérum et de glucose à une concentration inférieure à environ 30 mM ;
b. la prolifération des cellules dans le milieu pendant environ deux semaines pour obtenir une population de cellules enrichie en cellules stromales ; et
c. l'isolement de cellules qui se lient de façon spécifique à un agent spécifique d'un marqueur protéique spécifiquement exprimé dans les cellules stromales dérivées de cellules pancréatiques adultes, ce qui permet d'obtenir une population pure de cellules stromales dérivées de cellules pancréatiques adultes.

21. Procédé selon la revendication 12, 17 ou 20, dans lequel la concentration du sérum est inférieure à environ 5 %.

22. Procédé selon la revendication 19, dans lequel la population de cellules stromales dérivées de cellules pancréatiques adultes provenant d'un pancréas est multipliée en culture avant le traitement visant à induire la différenciation.

23. Procédé selon la revendication 19, dans lequel les cellules présentant les caractéristiques de la lignée de cellules β sont des cellules produisant de l'insuline.

24. Cellules stromales dérivées de cellules pancréatiques adultes selon l'une quelconque des revendications 1 à 6 pour utilisation dans le traitement d'un patient souffrant d'un diabète sucré ou risquant de développer un diabète, les cellules stromales étant obtenues par isolement d'une population de cellules stromales dérivées de cellules pancréatiques adultes provenant du tissu pancréatique d'un donneur, et les cellules stromales dérivées de cellules pancréatiques adultes se différenciant, suite à l'introduction des cellules stromales dérivées de cellules pancréatiques adultes chez le patient, en cellules produisant des hormones pancréatiques.

25. Cellules progénitrices pour utilisation dans le traitement d'un patient souffrant d'un diabète sucré ou risquant de développer un diabète, lesdites cellules progénitrices étant obtenues par un procédé *ex vivo* comprenant :
la multiplication *ex vivo* de cellules stromales ayant été isolées à partir du tissu pancréatique d'un donneur, les cellules étant négatives pour l'expression de NCAM, ABCG2, la cytokératine 7, la cytokératine 8, la cytokératine 18 et la cytokératine 19, et les cellules de la population étant positives pour l'expression d'au moins un marqueur protéique choisi dans le groupe constitué de CD44, CD73, CD90 ou CD105, pour produire des cellules progénitrices, et les cellules progénitrices se différenciant, suite à leur introduction chez le patient, en cellules produisant des hormones pancréatiques.

26. Cellules pour utilisation selon la revendication 24 ou 25, les cellules produisant des hormones pancréatiques étant des cellules produisant de l'insuline.

27. Cellules d'une lignée de cellules β pancréatiques pour utilisation dans le traitement d'un patient souffrant d'un diabète sucré ou risquant de développer un diabète, lesdites cellules d'une lignée de cellules β pancréatiques étant obtenues par un procédé *in vitro* comprenant :
(a) la multiplication *in vitro* d'une population de cellules stromales dérivées de cellules pancréatiques adultes ayant été isolées à partir du tissu pancréatique d'un donneur, les cellules étant négatives pour l'expression de NCAM, ABCG2, la cytokératine 7, la cytokératine 8, la cytokératine 18 et la cytokératine 19, et les cellules de la population étant positives pour l'expression d'au moins un marqueur protéique choisi dans le groupe constitué de CD44, CD73, CD90 ou CD105 ; et
(b) la différenciation des cellules stromales dérivées de cellules pancréatiques adultes multipliées en une lignée de cellules β pancréatiques.

28. Cellules pour utilisation selon la revendication 27, les cellules de la lignée de cellules β pancréatiques étant des cellules produisant de l'insuline.

29. Cellules pour utilisation selon la revendication 25, 26 ou 27, le patient étant le donneur du tissu pancréatique.

30. Cellules stromales dérivées de cellules pancréatiques adultes et îlots selon l'une quelconque des revendications 1 à 6 pour utilisation dans le traitement d'un patient souffrant d'un diabète sucré ou risquant de développer un diabète, les cellules stromales et les îlots étant obtenus par un procédé comprenant :
(a) l'isolement d'une population de cellules stromales dérivées de cellules pancréatiques adultes provenant du tissu pancréatique d'un donneur ; et
(b) l'obtention d'îlots matures auprès d'un donneur, et, suite à l'introduction des cellules stromales dérivées de cellules pancréatiques adultes et des îlots chez le patient :
(i) les îlots améliorant la survie et le fonctionnement des cellules stromales dérivées de cellules pancréatiques adultes chez le patient ; ou
(ii) les cellules stromales dérivées de cellules pancréatiques adultes améliorant la survie et le fonctionnement des îlots chez le patient.

31. Cellules pour utilisation selon la revendication 30, le donneur des îlots matures étant allogénique ou xénogénique par rapport au patient.

32. Cellules pour utilisation selon l'une quelconque des revendications 24, 25, 27 ou 30, les cellules devant être introduites chez le patient étant incorporées dans un matériau de support polymère.

33. Cellules pour utilisation selon la revendication 32, le matériau de support polymère étant également chargé d'un excipient pharmaceutique ou d'un agent bioactif qui facilite la survie et le fonctionnement des cellules chez le patient.
